(19) Europäisches Patentamt
European Patent Office
Office européen des brevets

(11) EP 4 559 573 A1

(12) **EUROPEAN PATENT APPLICATION**
published in accordance with Art. 153(4) EPC

(43) Date of publication:
**28.05.2025 Bulletin 2025/22**

(21) Application number: **23842888.2**

(22) Date of filing: **12.07.2023**

(51) International Patent Classification (IPC):
***B01J 23/44*** *(2006.01)* ***B01J 35/60*** *(2024.01)*
***C07C 209/36*** *(2006.01)* ***C07C 211/52*** *(2006.01)*
***C07B 61/00*** *(2006.01)*

(52) Cooperative Patent Classification (CPC):
**B01J 23/44; B01J 33/00; C07C 209/36; C07C 211/52;** C07B 61/00

(86) International application number:
**PCT/JP2023/025732**

(87) International publication number:
**WO 2024/018965 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.07.2022 JP 2022116750**

(71) Applicant: **Chiyoda Corporation**
**Kanagawa 220-8765 (JP)**

(72) Inventors:
- **KADO Shigeru**
  **Yokohama-shi, Kanagawa 220-8765 (JP)**
- **WATANABE Toshiyuki**
  **Yokohama-shi, Kanagawa 220-8765 (JP)**
- **KIRYU Asako**
  **Yokohama-shi, Kanagawa 220-8765 (JP)**
- **MAEDA Kyogo**
  **Yokohama-shi, Kanagawa 220-8765 (JP)**

(74) Representative: **Gulde & Partner**
**Patent- und Rechtsanwaltskanzlei mbB**
**Berliner Freiheit 2**
**10785 Berlin (DE)**

(54) **HYDROGENATION CATALYST, AND FLOW-TYPE ORGANIC SYNTHESIS SYSTEM AND METHOD FOR PRODUCING HYDROGENATED ORGANIC COMPOUND USING SAME**

(57) [Problem] To inhibit a reduction in selectivity of a halogenated aromatic amine as a target product caused by proceeding of a halogen elimination reaction in hydrogenation of an aromatic halonitro compound.

[Solution] A hydrogenation catalyst is used in hydrogenation of an aromatic halonitro compound. The hydrogenation catalyst includes a carrier including at least one of titania and alumina and palladium supported on the carrier. The palladium has an average particle diameter of 9.7 nm or less.

Fig.2

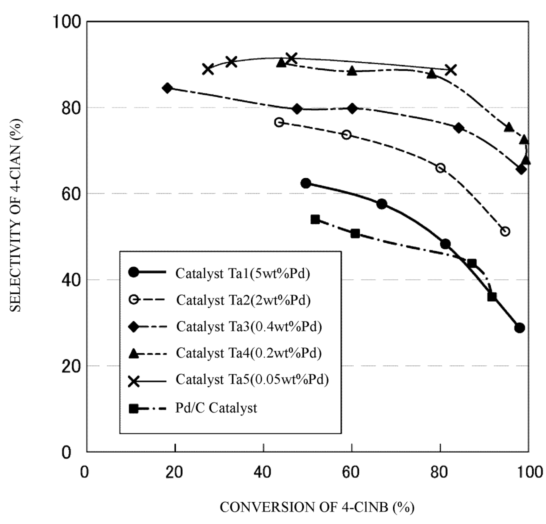


EP 4 559 573 A1

## Description

[Technical Field]

The present invention relates to a hydrogenation catalyst that is used for hydrogenation of an aromatic halonitro compound, and to a flow-type organic synthesis system, and a method for producing a hydrogenated organic compound, using the same.

[Background Art]

In hydrogenation where hydrogen is added to an unsaturated bond in an aromatic halonitro compound, a dehalogenation reaction (halogen elimination) readily occurs, and thus reduces the yield and quality of a halogenated aromatic amine as a target product.

As a conventional technique for inhibiting a dehalogenation reaction in hydrogenation of an aromatic halonitro compound, for example, a method of hydrogen reduction of the aromatic halonitro compound in the presence of an acidic phosphorus compound is known (refer to Patent Literature 1). Another known method is to hydrogenate an aromatic halonitro compound by use of an organic nitrogenous base (alkylamines, alicyclic amines, or guanidine) (refer to Patent Literature 2). Yet another known method is to produce halogenated anilines from halogenated nitrobenzenes by catalytic reduction in the coexistence with halogenated benzenes (refer to Patent Literature 3). Still another known method is to hydrogenate an aromatic halonitro compound by use of a hydrogenation catalyst in the presence of carbon dioxide (refer to Patent Literature 4). Also known is a hydrogenation catalyst that has a catalytic metal, such as platinum, supported on or impregnated in a carrier material including carbon activated by phosphoric acid or its salt (refer to Patent Literature 5).

[Citation List]

[Patent Literature]

[Patent Literature 1] JPS51-122029A
[Patent Literature 2] JPS52-35652B
[Patent Literature 3] JPH7-133255A
[Patent Literature 4] JP2004-277409A
[Patent Literature 5] JPS63-033902B

[Summary of Invention]

[Technical Problem]

The use of additives, gases, or the like (hereinafter referred to as "dehalogenation inhibitor") for inhibiting the dehalogenation reaction as in the conventional techniques described in Patent Literatures 1 to 5 requires a dehalogenation inhibitor recovery process. That complicates a product (target product) production process, and also leads to a risk that the dehalogenation inhibitor is mixed into the product. Furthermore, the conventional technique described in Patent Literature 4 increases the burden of safety control and product quality control in a production process where carbon dioxide is handled.

Further, hydrogenation of an aromatic halonitro compound (for example, halonitrobenzene) may produce as a by-product a nitroso compound (for example, nitrosobenzene) that is harmful to humans, aquatic creatures, and the like and that can burn explosively. It is therefore important to inhibit the production of the nitroso compound. In this regard, the inventors developed a hydrogenation catalyst that improves the yield of a halogenated aromatic amine as a target product in hydrogenation of an aromatic halonitro compound without the need for a dehalogenation inhibitor while inhibiting the production of a nitroso compound. The inventors have filed a patent application for the hydrogenation catalyst (Patent Application Number JP 2021-072144).

However, a hydrogenation reaction of an aromatic halonitro compound has a problem of selectivity of a target product (halogenated aromatic amine) being reduced by proceeding of a excessive reaction (halogen elimination reaction). Such a reduction in selectivity of the target product caused by the proceeding of the halogen elimination reaction is not particularly dealt with in the conventional techniques described in Patent Literatures 1 to 5.

The present invention is intended to solve the foregoing problems of the conventional techniques. An object of the present invention is to provide a hydrogenation catalyst that inhibits, in hydrogenation of an aromatic halonitro compound,

a reduction in selectivity of a halogenated aromatic amine as a target product caused by proceeding of a halogen elimination reaction, and to provide a flow-type organic synthesis system, and a method for producing a hydrogenated organic compound, using the same.

[Solution to Problem]

In a first aspect of the present invention, a hydrogenation catalyst that is used for hydrogenation of an aromatic halonitro compound includes: a carrier including at least one of titania and alumina; and palladium supported on the carrier. The palladium has an average particle diameter of 9.7 nm or less.

This inhibits, in hydrogenation of an aromatic halonitro compound, a reduction in selectivity of a halogenated aromatic amine as a target product caused by proceeding of a halogen elimination reaction.

In a second aspect of the present invention, the hydrogenation catalyst has a CO adsorption inhibition rate, indicated by the following formula, of 61% or less:

[Math. 1]

$$\text{CO adsorption inhibition rate (\%)} = \left(1 - \frac{D_{CO}}{D_{TEM}}\right) \times 100$$

where $D_{CO}$ is metal dispersion based on a CO pulse method, and $D_{TEM}$ is metal dispersion based on TEM (transmission electron microscope) observation.

The CO adsorption inhibition rate of the hydrogenation catalyst is adjusted within an appropriate range, thereby inhibiting a reduction in selectivity of a halogenated aromatic amine as a target product, in hydrogenation of an aromatic halonitro compound, while increasing conversion speed of the aromatic halonitro compound.

In a third aspect of the present invention, the carrier is a composite carrier consisting of alumina and titania, and the composite carrier includes a structure where a base material consisting of alumina is coated with titania.

This effectively improves the yield of a halogenated aromatic amine as a target product, in hydrogenation of an aromatic halonitro compound, while effectively inhibiting the production of a nitroso compound.

In a fourth aspect of the present invention, the carrier including titania includes titania with 38% or more anatase crystal structure.

This effectively improves the yield of a halogenated aromatic amine as a target product while effectively inhibiting the production of a nitroso compound in a case where a hydrogenation catalyst with a carrier including titania is used in hydrogenation of an aromatic halonitro compound.

In a fifth aspect of the invention, the carrier including titania does not include a rutile crystal structure.

This effectively improves the yield of a halogenated aromatic amine as a target product while effectively inhibiting the production of a nitroso compound in a case where a hydrogenation catalyst with a carrier including titania is used in hydrogenation of an aromatic halonitro compound.

In a sixth aspect of the present invention, the carrier including titania has an average pore diameter that is 78 times or less as long as longitudinal molecular length of the aromatic halonitro compound.

This effectively improves the yield of a halogenated aromatic amine as a target product, in hydrogenation of an aromatic halonitro compound, while effectively inhibiting the production of a nitroso compound.

In a seventh aspect of the present invention, the carrier has an average pore diameter of approximately 50 nm or less.

This effectively improves the yield of a halogenated aromatic amine as a target product, in hydrogenation of an aromatic halonitro compound, while effectively inhibiting the production of a nitroso compound.

In an eighth aspect of the present invention, a flow-type organic synthesis system is configured to initiate a gas-liquid-solid three-phase reaction by using: a fixed catalyst including the hydrogenation catalyst; hydrogen as a gas raw material; and an aromatic halonitro compound as a liquid raw material.

This inhibits, in hydrogenation of an aromatic halonitro compound, a reduction in selectivity of a halogenated aromatic amine as a target product caused by proceeding of a halogen elimination reaction while controlling reaction temperature more precisely as compared with a batch method.

In a ninth aspect of the present invention, a method for producing a hydrogenated organic compound is provided in which the hydrogenation catalyst is used to hydrogenate the aromatic halonitro compound.

This inhibits, in hydrogenation of an aromatic halonitro compound, a reduction in selectivity of a halogenated aromatic amine as a target product caused by proceeding of a halogen elimination reaction.

In a tenth aspect of the present invention, the hydrogenation catalyst is used to hydrogenate the aromatic halonitro compound without being activated at or above 250 °C.

This allows hydrogenation of an aromatic halonitro compound, while simplifying a production process of a hydrogenated organic compound, through the use of an appropriate hydrogenation catalyst.

[Advantageous Effects of Invention]

According to the present invention, thus, a reduction in selectivity of a halogenated aromatic amine as a target product caused by proceeding of a halogen elimination reaction is inhibited in hydrogenation of an aromatic halonitro compound.

[Brief Description of Drawings]

Fig. 1 is a diagram showing an overall configuration of a flow-type organic synthesis system that is used for hydrogenation of an aromatic halonitro compound according to an embodiment.
Fig. 2 is a graph showing the relationship between the conversion of 4-ClAB as a liquid raw material and the selectivity of 4-ClAN in a hydrogenation reaction (for catalysts Ta1 to Ta5).
Figs. 3A to 3D are diagrams showing examples of images obtained by TEM (for catalysts Ta1 to Ta2 and Ta4 to Ta5).
Fig. 4 is a graph showing the relationship between residence time and 4-ClAN consumption rate per atom of palladium exposed at a particle surface (for average particle diameters 9.7 nm, 7.0 nm, and 1.4 nm).
Fig. 5 is a graph showing the relationship between CO adsorption inhibition rate and 4-ClAB consumption rate per atom of palladium exposed at a particle surface (for catalysts Ta1 to Ta2 and Ta4 to Ta5).
Fig. 6 is a graph showing the relationship between Pd loading and Pd dispersion (for catalysts Ta1 to Ta5).
Fig. 7 is a graph showing the relationship between Pd loading and Pd particle diameter (for catalysts Ta1 to Ta5).
Fig. 8 is a graph showing the relationship between the conversion of 4-ClAB as a liquid raw material and the selectivity of 4-ClAN in a hydrogenation reaction (for catalysts Ta4, Tb1, S1, A1, and H1).
Fig. 9A is a graph showing the relationship between the conversion of 4-ClAB and the selectivity of 4-ClAN, and Fig. 9B is a graph showing the relationship between the conversion of 4-ClAB and the selectivity of a by-product (for catalysts Ta2 and Ta4).
Fig. 10A is a graph showing the relationship between the conversion of 4-ClAB and the selectivity of 4-ClAN, and Fig. 10B is a graph showing the relationship between the conversion of 4-ClAB and the selectivity of a by-product (for catalysts A1 and A2).
Fig. 11A is a graph showing the relationship between the conversion of 4-ClAB and the selectivity of 4-ClAN, and Fig. 11B is a graph showing the relationship between the conversion of 4-ClAB and the selectivity of a by-product (for catalysts H1 and H2).
Fig. 12A is a graph showing the relationship between the conversion of 4-ClAB and the selectivity of 4-ClAN, and Fig. 12B is a graph showing the relationship between the conversion of 4-ClAB and the selectivity of a by-product (for catalysts Tc1 and Tc2).
Fig. 13A is a graph showing the relationship between the conversion of 4-ClAB and the selectivity of 4-ClAN, and Fig. 13B is a graph showing the relationship between the conversion of 4-ClAB and the selectivity of a by-product (for catalysts Tb1 and Tb2).
Fig. 14A is a graph showing the relationship between the conversion of 4-ClAB and the selectivity of 4-ClAN, and Fig. 14B is a graph showing the relationship between the conversion of 4-ClAB and the selectivity of a by-product (for catalysts S1 and S2).
Fig. 15 is a graph showing the relationship between the conversion of 4-ClAB and the selectivity of a nitroso compound in a hydrogenation reaction (for catalyst S2, catalyst Td1, catalyst A2, and catalyst H2).
Fig. 16 is a graph showing the relationship between the conversion of 4-ClAB and the selectivity of 4-ClAN in a hydrogenation reaction (for catalyst S2, catalyst Td1, catalyst A2, and catalyst H2).
Fig. 17 is a graph showing the relationship between the conversion of 4-ClAB and the selectivity of a nitroso compound in a hydrogenation reaction (for catalyst Ta2);
Fig. 18 is a graph showing the relationship between the conversion of 4-ClAB and the selectivity of 4-ClAN in a hydrogenation reaction (for catalyst Ta2).
Fig. 19 is a graph showing a X-ray diffraction pattern based on an X-ray diffraction method (XRD) (for catalyst Ta2).
Fig. 20 is a graph showing the effects of average pore diameters of carriers including titania on the action of respective catalysts using the carriers (for catalyst Td1, catalyst Ta2, and catalyst Te1).
Fig. 21 is a graph showing the effects of the average pore diameters of the carriers including titania on the action of the respective catalysts using the carriers (for catalyst Td1, catalyst Ta2, and catalyst Te1).

[Description of Embodiments]

Described below with reference to drawings are a hydrogenation catalyst according to embodiments, and a flow-type organic synthesis system, and a method for producing a hydrogenated organic compound, using the hydrogenation catalyst.

(Hydrogenation Catalyst)

The hydrogenation catalyst according to an embodiment includes a carrier including at least one of titania ($TiO_2$) and alumina ($Al_2O_3$), and at least one metal (hereinafter referred to as "catalytic metal") that is selected from the group 10 elements in the periodic table and that is supported on the carrier.

As the catalytic metal, nickel (Ni), palladium (Pd), platinum (Pt), or the like included in the group 10 elements in the periodic table can be used, and palladium is particularly preferable. A precursor of palladium, for example, includes, but is not limited to, palladium compounds such as its nitrate, sulfate, chloride, and acetylacetone complex. The catalytic metal is dispersed and supported on an outer surface of the carrier and on inner surfaces of pores of the carrier.

Palladium as the catalytic metal preferably has an average particle diameter of 9.7 nm or less. This inhibits, in hydrogenation of an aromatic halonitro compound, a reduction in selectivity of a halogenated aromatic amine as a target product caused by proceeding of a halogen elimination reaction.

The shape and size (average particle size or the like) of a base material such as titania and alumina included in the carrier are not specifically limited, and various shapes and sizes can be adopted depending on the specifications of a reactor used in a hydrogenation reaction of an aromatic halonitro compound, reaction conditions, and the like. As the base material consisting of at least one of titania and alumina, a commercially available base material for a catalytic carrier can be used. An example of commercially available titania for a catalytic carrier is the CS-300S-12 series and CS-950-12 series manufactured by Sakai Chemical Industry Co., Ltd.. An example of commercially available alumina for a catalytic carrier is the Neobead GB series manufactured by Mizusawa Industrial Chemicals, Ltd..

As the base material consisting of at least one of titania and alumina, a material (porous inorganic oxide) can be used that is obtained by synthesizing a hydrosol or hydrogel of a hydrous metal oxide by pH swing in which the hydrosol or hydrogel is swung alternately multiple times between a precipitation pH region and a dissolution pH region thereof.

The hydrous metal oxide is a hydrous oxide of one or more metals selected from titanium (Ti) and aluminum (Al). For a metal compound as a raw material for synthesizing the hydrosol or hydrogel of the hydrous metal oxide, for example, a chloride, a fluoride, a bromide, an iodide, salts such as nitrate, sulfate, carbonate, acetate, phosphate, borate, oxalate, hydrofluoric acid salt, silicate, and iodate, oxoacid salt, alkoxides, and the like, of the metal or metals can be used. One kind of metal compound may be used singularly, or a plurality of kinds of metal compounds may be used as a mixture thereof.

Examples of usable titanium compounds are titanium tetrachloride ($TiCl_4$), Titanium sulfate [$Ti_2(SO_4)_3$, $Ti(SO_4)_2$], titanium oxysulfate ($TiOSO_4$), titanium trichloride ($TiCl_3$), titanium bromide ($TiBr_4$), titanium fluoride ($TiF_4$, $TiF_3$), titanium oxide ($TiO_2$), orthotitanic acid ($H_4TiO_4$), metatitanic acid ($H_2TiO_3$), titanium methoxide [$Ti(OCH_3)_4$], titanium ethoxide [$Ti(OC_2H_5)_4$], titanium propoxide [$Ti(OC_3H_7)_4$], titanium isopropoxide {$Ti[OCH(CH_3)_2]_4$}, titanium butoxide [$Ti(OC_4H_9)_4$], and the like.

Examples of usable aluminum compounds are metal aluminum (Al), aluminum chloride ($AlCl_3$, $AlCl_3 \cdot 6H_2O$), aluminum nitrate [$Al(NO_3)_3 \cdot 9H_2O$], aluminum sulfate [$Al_2(SO_4)_3$, $Al_2(SO_4)_3 \cdot 18H_2O$], polyaluminum chloride [$(Ab(OH) \cdot Cl_{6-n})_m$(1<n<5, m<10)], ammonium alum [$NH_4Al(SO_4)_2 \cdot 12H_2O$], sodium aluminate ($NaAlO_2$), potassium aluminate ($KAlO_2$), aluminum isopropoxide [$Al[OCH(CH_3)_2]_3$], aluminum ethoxide [$Al(OC_2H_5)_3$], aluminum-t-butoxide [$Al[OC(CH_3)_3]_3$], aluminum hydroxide [$Al(OH)_3$], and the like.

For details of the above-described carrier synthesized by the pH swing and its manufacturing method, refer to a porous inorganic oxide, and its manufacturing method, as disclosed in Japanese Patent No. 4119144, for example, the disclosure of which is herein incorporated by reference. The carrier synthesized by the pH swing allows pore diameter (average or distribution) of the catalyst to be controlled to a desired value by the pH swing. Also, the carrier allows fine adjustment of the value of the pore diameter by adjustment of a calcination temperature of the carrier at the time of calcination.

Further, a composite carrier consisting of alumina and titania may be used as the carrier. The composite carrier includes a structure that a base material consisting of alumina is coated with titania. As the shape of the composite carrier, for example, a skeleton structure can be used in which a large number of pores are formed by a plurality of needle-like or columnar bodies being three-dimensionally intertwined. This structure allows an increase in specific surface area (ratio of pore volume to pore diameter) of the hydrogenation catalyst, and facilitates control of the pore structure.

For details of the above-described composite carrier, refer to the carrier and its manufacturing method as disclosed in Japanese Patent No. 6456204, for example, the disclosure of which is herein incorporated by reference. The composite carrier allows production of a catalyst, with a relatively small amount of the catalytic metal, that excels in stability and suppression of side reactions.

The carrier including titania preferably includes titania with 38% or more anatase crystal phase (crystal structure). This stably improves the yield of a halogenated aromatic amine as a target product while effectively inhibiting the production of a nitroso compound. Also, the carrier more preferably includes titania with 96% or more anatase crystal phase, and still more preferably includes titania with 100% anatase. The percentage of the anatase crystal phase can be changed by changing the calcination temperature of the carrier. Specifically, the carrier including titania preferably includes 38% or more anatase crystal phase, and does not include a rutile crystal phase. Further, the crystal phases of the carrier can be identified through X-ray diffraction analysis using an X-ray diffraction device.

The carrier including titania preferably has an average pore diameter of approximately 50 nm or less. This stably improves the yield of a halogenated aromatic amine as a target product while effectively inhibiting the production of a nitroso compound. Further, the carrier including titania more preferably has an average pore diameter of approximately 18 nm or less, and still more preferably of approximately 7 nm or less.

Also, the carrier including titania preferably has an average pore diameter that is 78 times or less as long as longitudinal molecular length of the aromatic halonitro compound. The average pore diameter of the carrier including titania is more preferably 28 times or less as long as the longitudinal molecular length of the aromatic halonitro compound, and is still more preferably 11 times or less as long as the same.

The average pore diameter of the carrier can be measured using a pore distribution measuring device (for example, a mercury porosimeter). The longitudinal molecular length of the aromatic halonitro compound can be calculated using a quantum chemical calculation program.

The processes as described in the foregoing patent literatures or known catalyst production processes (for example, an impregnation process, a drying process, a calcination process, a reduction process, or the like) can be adopted appropriately to production of the above-described hydrogenation catalyst with the catalytic metal supported on the carrier. Also, the catalyst (or the carrier) can be formed into a desired shape such as a spherical shape, a columnar shape, a cylindrical shape, and the like depending on the intended use. Further, the size (particle size or the like) of the catalyst (or the carrier) can be set appropriately.

(Hydrogenation of Aromatic Halonitro Compound)

Below described is a hydrogenation reaction of an aromatic halonitro compound using a hydrogenation catalyst according to an embodiment.

The hydrogenation of the aromatic halonitro compound is represented by the following general Formula (1).

[Chem. 1]

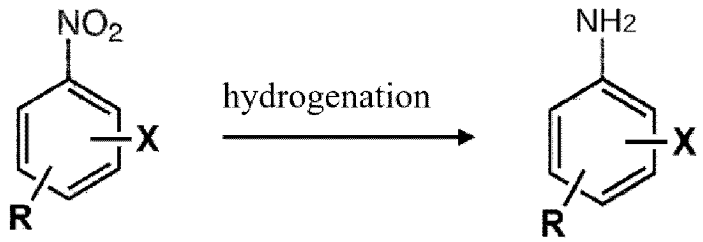


(1)

In Formula (1), X represents fluorine, chlorine, bromine, or iodine. R represents a hydrogen atom, a hydroxyl group, a carboxyl group, a sulfo group, an alkyl group, an alkenyl group, an aralkyl group, an aryl group, an acyl group, an aroyl group, an alkoxy group, or an alkoxycarbonyl group.

Hydrogenation of 4-chloronitrobenzene is described below as an example of the hydrogenation of the aromatic halonitro compound. In the hydrogenation of 4-chloronitrobenzene, as shown in the process of Formula (2), hydrogen is added to 4-chloronitrobenzene as a raw material to produce 4-chloroaniline as a target product via 4-chloronitrosobenzene and 4-chlorophenylhydroxylamine as intermediates. 4-chloroaniline undergoes a halogen elimination reaction to produce aniline. The excessive reaction proceeds, thereby reducing the selectivity of 4-chloroaniline.

On the other hand, 4-chloronitrobenzene can be dehalogenated to produce nitrobenzene. Nitrobenzene is hydrogenated to produce aniline via nitrosobenzene and hydroxylamine as intermediates. In the hydrogenation reaction according to the present embodiment, only 4-chloronitrosobenzene was detected as an intermediate, and nitrosobenzene was detected at negligible level.

[Chem. 2]

[Chem. 2]

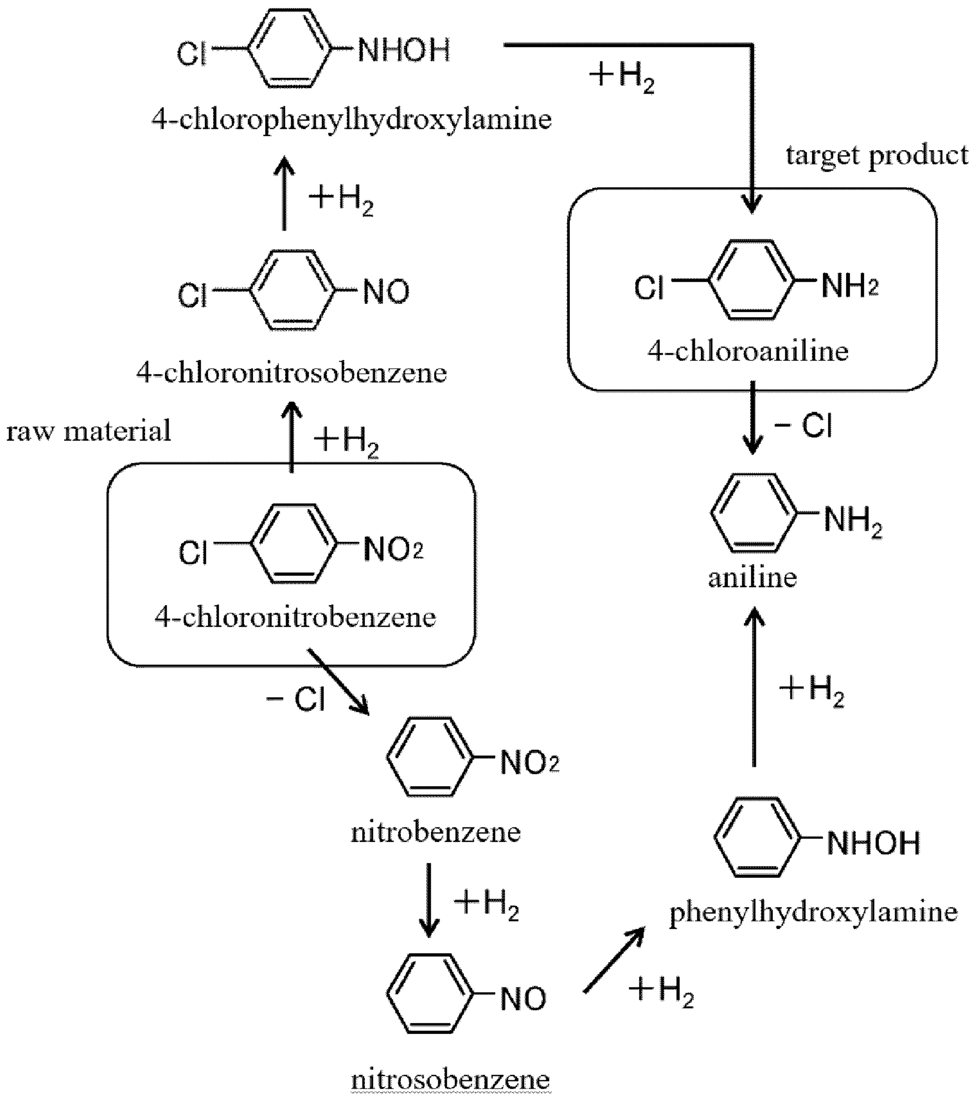


(2)

4-chloronitrobenzene can be hydrogenated using any of a batch type, a semi-batch type, and a flow-type, and the hydrogenation catalyst according to the present embodiment is particularly suitable as a flow-type reaction catalyst used in a flow-type reaction system that initiates a gas-liquid-solid three-phase reaction.

An example of the configuration of a flow-type organic synthesis system 1 (hydrogenation reaction system) is described below with reference to Fig. 1.

The flow-type organic synthesis system 1 includes a flow-type fixed-bed reactor 2 (hereinafter referred to as reactor 2) that initiates a gas-liquid-solid three-phase reaction by using hydrogen as a gas raw material, 4-chloronitrobenzene as a liquid raw material, and the hydrogenation catalyst as a solid catalyst. For the liquid raw material, an organic solvent (alcohol, ether, other aromatic hydrocarbons, or the like) that is inert to the reaction can be used. In a case where the aromatic halonitro compound to be hydrogenated is a liquid, the reaction can be initiated without a solvent.

The reactor 2 is a known tubular reactor accommodating a catalyst layer 3 including the hydrogenation catalyst. The reactor 2 initiates a reaction by causing the gas raw material and the liquid raw material, which are continuously supplied from an inlet line L1, to flow into the catalyst layer 3. Also, the reactor 2 is provided with a tubular electric furnace 4 consisting of two independent blocks, and allows adjustment of reaction temperature (more specifically, the temperature of the catalyst layer 3) by supplying heat as required. A usable example of the reactor 2 is a down-flow reactor that causes the raw materials to flow in the same direction as gravity, as well as an up-flow reactor that causes the raw materials to flow in the opposite direction to gravity can be used as the reactor 2.

The gas raw material is continuously supplied to the reactor 2 via a gas raw material supply line L2 connected to the inlet line L1. Also connected to the inlet line L1 is a purge gas supply line L3 through which a purge gas flows. The flow-type organic synthesis system 1 allows purging of devices, piping, or the like therein by supplying purge gas to the purge gas supply line L3 on occasions such as during maintenance.

The liquid raw material is stored in a liquid raw material tank 11. A liquid raw material pump 12 causes the liquid raw material to flow into the inlet line L1 via a liquid raw material supply line L4, and the liquid raw material is continuously supplied to the reactor 2 together with the gas raw material. The liquid raw material supply line L4 is provided with a preheater/precooler 13. The preheater/precooler 13 heats or cools the liquid raw material, thereby adjusting the temperature of the liquid raw material supplied to the reactor 2 within a preset target range.

From the reactor 2, a reaction product mainly including 4-chloroaniline is continuously discharged through an outlet line L6. The outlet line L6 is provided with a heat exchanger 21. The heat exchanger 21 heats or cools the reaction product, thereby adjusting the temperature of the reaction product as discharged from the reactor 2 within a preset target range.

The reaction product cooled by the heat exchanger 21 is supplied to a main drum 25 (gas-liquid separator) connected to a downstream portion of the outlet line L6. In the main drum 25, the reaction product is separated into off-gas (residual gas) including an unreacted gas raw material and the like and a recovery liquid including a target (product) of the reaction.

The off-gas (gas-phase component) as separated in the main drum 25 is discharged via an off-gas transportation line L7 (a first line of a residual gas transportation line).

The recovery liquid from the main drum 25 is supplied to a product recovery drum 41 via a recovery liquid transportation line L9 (target recovery line). In the product recovery drum 41, the gas remaining in the recovery liquid is separated from the recovery liquid. This separated gas is discharged to the outside via a separated gas discharge line L10. Also, the recovery liquid (target product) as separated in the product recovery drum 41 is recovered via a product recovery line L11.

In the flow-type organic synthesis system 1, an operator samples the recovery liquid from the product recovery line L11 appropriately at prescribed timings. The operator analyzes the recovery liquid using liquid chromatograph or gas chromatograph, thereby identifying and quantifying 4-chloroaniline or the like as a target product (product).

[Examples]

In the flow-type organic synthesis system 1 (refer to Fig. 1), an experiment was conducted to produce 4-chloroaniline (hereinafter referred to as 4-ClAN) as a target product by hydrogenating 4-chloronitrobenzene (hereinafter referred to as 4-ClAB) using each of the following catalysts Ta1 to Ta5, Tc1 to Tc2, Td1, Te1, A1 to A2, H1 to H2, and S1 to S2 as a hydrogenation catalyst. In the present embodiment, a commercially available Pd/C (palladium carbon) catalyst ("5% Palladium on Activated Carbon, Degussa type E 106 R/W 5% Pd (wetted with ca.55% water)" manufactured by Wako Pure Chemical Industries, Ltd.) with a metal loading of 5 wt. % was used as a catalyst (hereinafter referred to as "Pd/C catalyst") to be compared with the hydrogenation catalyst.

(Catalyst Ta1)

A commercially available titania ("CS-300S-12" manufactured by Sakai Chemical Industry Co., Ltd., crystal type: 100% anatase) for a catalytic carrier was calcined at 500 °C for 3 hours. 5 g of the calcined titania was weighed, impregnated with a 1.06% palladium nitrate aqueous solution [$Pd(NO_3)aq$] in such a manner as to have a Pd loading of 5 wt. % as theoretical value, dried, and then calcined at 500 °C for 3 hours. Thus, a catalyst Ta1 (Pd-supported titania catalyst) was obtained. Palladium in the catalyst Ta1 had an average particle diameter of 9.7 nm. As will be described later, the average particle diameter was calculated based on observation of images (TEM images) obtained by TEM (transmission electron microscope, "JEM-ARM-200F" manufactured by JEOL Ltd.) (as with other catalysts).

(Catalyst Ta2)

Titania for a catalytic carrier, similar to that in the catalyst Ta1, was used. By a method similar to that used for the catalyst Ta1, titania was impregnated with a 0.42% palladium nitrate aqueous solution [$Pd(NO_3)aq$] in such a manner as to have a Pd loading of 2 wt. %, dried, and then calcined at 500 °C for 3 hours. Thus, a catalyst Ta2 (Pd-supported titania catalyst) was obtained. Palladium in the catalyst Ta2 had an average particle diameter of 7.0 nm.

(Catalyst Ta3)

Titania for a catalytic carrier, similar to that in the catalyst Ta1, was used. By a method similar to that used for the catalyst Ta1, titania was impregnated with a 0.08% palladium nitrate aqueous solution [$Pd(NO_3)aq$] in such a manner as to have a Pd loading of 0.4 wt. %, dried, and then calcined at 500 °C for 3 hours. Thus, a catalyst Ta3 (Pd-supported titania

catalyst) was obtained.

(Catalyst Ta4)

Titania for a catalytic carrier, similar to that in the catalyst Ta1, was used. By a method similar to that used for the catalyst Ta1, titania was impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst Ta4 (Pd-supported titania catalyst) was obtained. Palladium in the catalyst Ta4 had an average particle diameter of 1.4 nm.

(Catalyst Ta5)

Titania for a catalytic carrier, similar to that in the catalyst Ta1, was used. By a method similar to that used for the catalyst Ta1, titania was impregnated with a 0.01% palladium nitrate aqueous solution [Pd($NO_3$)aq] in such a manner as to have a Pd loading of 0.05 wt. %, dried, and then calcined at 500 °C for 3 hours. Thus, a catalyst Ta5 (Pd-supported titania catalyst) was obtained. Palladium in the catalyst Ta5 had an average particle diameter of 1.5 nm.

(Catalyst Tb1)

A commercially available titania ("CS-300S-12" manufactured by Sakai Chemical Industry Co., Ltd., crystal type: 100% anatase) for a catalytic carrier was calcined at 950 °C for 3 hours. 5 g of the calcined titania was weighed, impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst Tb1 (Pd-supported titania catalyst) was obtained. Palladium in the catalyst Tb1 had an average particle diameter of 5.8 nm.

(Catalyst Tb2)

Titania for a catalytic carrier, similar to that in the catalyst Tb1, was used. By a method similar to that used for the catalyst Tb1, titania was impregnated with a 0.42% palladium nitrate aqueous solution [Pd($NO_3$)aq] in such a manner as to have a Pd loading of 2 wt. %, dried, and then calcined at 500 °C for 3 hours. Thus, a catalyst Tb2 (Pd-supported titania catalyst) was obtained. Palladium in the catalyst Tb2 had an average particle diameter of 17.9 nm.

(Catalyst Tc1)

A commercially available titania ("CS-300S-12" manufactured by Sakai Chemical Industry Co., Ltd., crystal type: 100% anatase) for a catalytic carrier was calcined at 500 °C for 3 hours. 5 g of the calcined titania was weighed, impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst Tc1 (Pd-supported titania catalyst) was obtained. Palladium in the catalyst Tc1 had an average particle diameter of 3.2 nm.

(Catalyst Tc2)

Titania for a catalytic carrier, similar to that in the catalyst Tc1, was used. By a method similar to that used for the catalyst Tc1, titania was impregnated with a 0.42% palladium nitrate aqueous solution [Pd($NO_3$)aq] in such a manner as to have a Pd loading of 2 wt. %, dried, and then calcined at 500 °C for 3 hours. Thus, a catalyst Tc2 (Pd-supported titania catalyst) was obtained. Palladium in the catalyst Tc2 had an average particle diameter of 7.2 nm.

(Catalyst Td1)

A titania carrier prepared by pH swing was calcined at 500 °C for 3 hours. 5 g of the calcined titania carrier was weighed, impregnated with a 0.42% palladium nitrate aqueous solution [Pd($NO_3$)aq] in such a manner as to have a Pd loading of 2.0 wt. %, dried, and then calcined at 500 °C for 3 hours. Thus, a catalyst Td1 (Pd-supported titania catalyst) was obtained. The titania carrier prepared by the pH swing was produced by a known method similar to the production method of a titania carrier disclosed in Japanese Patent No. 5599212 (refer to paragraph [0123]).

(Catalyst Te1)

A commercially available titania ("CS-950-12" manufactured by Sakai Chemical Industry Co., Ltd.) for a catalytic carrier was calcined at 500 °C for 3 hours. 5 g of the calcined titania was weighed, impregnated with a 0.42% palladium

nitrate aqueous solution [Pd($NO_3$)aq] in such a manner as to have a Pd loading of 2 wt. %, dried, and then calcined at 500 °C for 3 hours. Thus, a catalyst Te1 (Pd-supported titania catalyst) was obtained.

(Catalyst A1)

An alumina carrier prepared by pH swing was calcined at 500 °C for 3 hours. 5 g of the calcined alumina carrier was weighed, impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst A1 (Pd-supported alumina catalyst) was obtained. The alumina carrier prepared by the pH swing was produced by a known method similar to the production method of an alumina carrier disclosed in Japanese Patent No. 5599212 (refer to AS-2). Palladium in the catalyst A1 had an average particle diameter of 3.1 nm.

(Catalyst A2)

An alumina carrier, similar to that in the catalyst A1, was used. By a method similar to that used for the catalyst A1, the alumina carrier was impregnated with a 0.42% palladium nitrate aqueous solution [Pd($NO_3$)aq] in such a manner as to have a Pd loading of 2 wt. %, dried, and then calcined at 500 °C for 3 hours. Thus, a catalyst A2 (Pd-supported titania catalyst) was obtained.

(Catalyst H1)

A titania-coating alumina carrier (HBT) prepared by pH swing was calcined at 500 °C for 3 hours. 5 g of the calcined titania-coating alumina carrier was weighed, impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst H1 (Pd-supported titania-coating alumina catalyst) was obtained. The titania-coating alumina carrier (HBT) prepared by the pH swing was produced by a known method similar to the production method of a titania-coating alumina carrier disclosed in Japanese Patent No. 5599212 (refer to AT-2). Palladium in the catalyst H1 had an average particle diameter of 2.7 nm.

(Catalyst H2)

A titania-coating alumina carrier, similar to that in the catalyst H1, was used. By a method similar to that used for the catalyst H1, the titania-coating alumina carrier was impregnated with a 0.42% palladium nitrate aqueous solution [Pd($NO_3$)aq] in such a manner as to have a Pd loading of 2 wt. %, dried, and then calcined at 500 °C for 3 hours. Thus, a catalyst H2 (Pd-supported titania-coating alumina catalyst) was obtained.

(Catalyst S1)

A commercially available silica gel ("CARiACT Q-10" manufactured by Fuji Silysia Chemical Ltd.) for a catalytic carrier was calcined at 500 °C for 3 hours. 5 g of the calcined silica gel was weighed, impregnated with a 0.04% palladium nitrate aqueous solution [Pd($NO_3$)aq] in such a manner as to have a Pd loading of 0.2 wt. %, dried, and then calcined at 500 °C for 3 hours. Thus, a catalyst S1 (Pd-supported silica catalyst) was obtained. Palladium in the catalyst S1 had an average particle diameter of 3.5 nm.

(Catalyst S2)

Silica gel for a catalytic carrier, similar to that in the catalyst S1, was used. By a method similar to that used for the catalyst Tc1, the silica gel was impregnated with a 0.42% palladium nitrate aqueous solution [Pd($NO_3$)aq] in such a manner as to have a Pd loading of 2 wt. %, dried, and then calcined at 500 °C for 3 hours. Thus, a catalyst S2 (Pd-supported Silica catalyst) was obtained. Palladium in the catalyst S2 had an average particle diameter of 5.5 nm.

In the hydrogenation reaction of 4-chloronitrobenzene using the flow-type organic synthesis system 1, a stainless steel reactor tube in the reactor 2 was filled with 1.0 g of the hydrogenation catalyst, and then the catalyst layer 3 was placed in such a manner that the center thereof was arranged on a second stage (upper stage) of the tubular electric furnace 4 consisting of the two independent blocks. Next, the catalyst layer 3 was heated in such a manner that the temperature of the center thereof reached 40 °C. Subsequently, 4-ClAB/TOL-IPA (toluene isopropyl alcohol solvent) as a liquid raw material was supplied to the reaction tube at a flow rate of 0.2 ml/min by use of the liquid raw material pump 12, and hydrogen as a gas raw material was supplied to the reaction tube at a flow rate of 20 $cm^3$/min. With the initiation of the hydrogenation reaction (reduction reaction) of the liquid raw material, the temperature of the hydrogenation catalyst

increases according to the heat generated by the liquid raw material. Accordingly, the temperature at the center of the catalyst layer 3 during the reaction was controlled at 40 °C by adjusting the temperature of each block of the electric furnace 4 during the reaction. After the reaction, the product was cooled, recovered, and analyzed by gas chromatography. Also, the flow rate of an outflow gas after cooling the product was measured by a mass flowmeter. From the analysis and measurement results, the conversion of 4-ClAB, the selectivity of 4-ClAN (target product), and the selectivity of 4-chloronitrosobenzene (nitroso compound) were calculated. Such an experiment on the hydrogenation reaction of 4-chloronitrobenzene was conducted for each of the foregoing hydrogenation catalysts and the Pd/C catalyst.

Fig. 2 is a graph showing the relationship between the conversion of 4-ClAB as a liquid raw material and the selectivity of 4-ClAN in the hydrogenation reaction for the catalysts Ta1 to Ta5 and the Pd/C catalyst (catalyst for comparison). Figs. 3 are diagrams showing examples of images obtained by TEM for catalysts Ta1 to Ta2 and Ta4 to Ta5. Fig. 4 is a graph showing the relationship between residence time (duration of contact between a reacting fluid and the hydrogenation catalysts in the reactor 2) and 4-ClAN consumption (conversion) rate per atom of palladium exposed at a particle surface for respective particle diameters of the catalysts.

Fig. 2 confirms that the selectivity of 4-ClAN exhibited by each of the catalysts Ta1 to Ta5 A is basically higher than that exhibited by the Pd/C catalyst for the conversion of 4-ClAB of a similar value. More specifically, PD-supported titania catalysts with a Pd loading of 5 wt. % or less (4.34 wt. % or less as measured value by ICP analysis to be described later) have higher selectivity of 4-ClAN (target product) compared to the commonly used Pd/C catalyst. In the PD-supported titania catalysts, further, the selectivity of 4-ClAN increases as Pd loading decreases from the catalyst Ta1 to the catalyst Ta5. To avoid a decrease in catalytic activity caused by a decrease in palladium to be involved in the hydrogenation reaction, however, Pd loading is preferably 0.05 wt. % or more, and more preferably 0.2 wt. % (0.17 wt. % as measured value by ICP analysis to be described later) or more.

As shown in Figs.3A to 3D, observation of TEM images (i.e. particle diameters calculated from TEM images) confirms that the size (average particle diameter) of palladium supported on the catalysts basically decreases as Pd loading decreases from the catalyst Ta1 to the catalyst Ta5.

Table 1 illustrates analysis results of the catalysts Ta1 to Ta2 and Ta4 to Ta5 obtained from the observation of TEM images.

[Table 1]

| | Pd loading (theoretical value) (wt%) | Pd loading (ICPmeasured value) (wt%) | Average particle diameter (nm) | Surface metal atom number (quantity/g) | Pd dispersion (%) |
|---|---|---|---|---|---|
| Catalyst T a 1 | 5 | 4.34 | 9.7 | $2.8\times10^{19}$ | 11.6 |
| Catalyst T a 2 | 2 | 1.77 | 7 | $1.6\times10^{19}$ | 16 |
| Catalyst T a 4 | 0.2 | 0.17 | 1.4 | $0.77\times10_{19}$ | 80.1 |
| Catalyst T a 5 | 0.05 | 0.05 | 1.5 | $0.20\times10_{19}$ | 74.7 |

Table 1 indicates Pd loading (wt. %), average particle diameter (nm), surface metal atom number (number/g), and PD dispersion (%) for each of the catalysts Ta1 to Ta2 and Ta4 to Ta5. As Pd loading (wt. %) indicated are theoretical values prior to catalyst preparation and measured values, after catalyst preparation, by ICP (Inductively Coupled Plasma) analysis. The average particle diameter (nm) is length mean diameter obtained from direct measurement of diameters (equivalent circle diameters) of a plurality of particles of catalyst metal (palladium here) present in a plurality of images. With regard to particles with elliptic shapes far from a circular shape, in particular those with a ratio of major axis to minor axis thereof of 1.5 or more, the average particle diameter is a value obtained by adding and dividing by 2 the lengths of the major and minor axes. By a similar method, average particle diameters of the other catalysts are calculated based on TEM images. Surface metal atom number (number/g) is the number of catalyst metal atoms exposed at a particle surface. PD dispersion is obtained by a method to be described later.

When the results as shown above in Fig. 2 are taken into consideration, it can be seen that the PD-supported titania catalysts with average palladium particle diameters of 9.7 nm or less have higher selectivity of 4-ClAN (target product) compared to the commonly used Pd/C catalyst. For higher selectivity of 4-ClAN than the commonly used Pd/C catalyst, more specifically, the PD-supported titania catalysts have average palladium particle diameters of 9.7 nm or less. Also, it can be seen that, in the PD-supported titania catalysts, the selectivity of 4-ClAN increases as the average palladium particle diameters decrease from the catalyst Ta1 to the catalyst Ta5.

As indicated in Fig. 4, it can be seen that, for residence time of a similar value, 4-ClAN consumption rate decreases (i.e., a halogen elimination reaction (excessive reaction) can be inhibited) as the average palladium particle

diameters in the PD-supported titania catalysts decrease.

Fig. 5 is a graph showing the relationship between CO adsorption inhibition rate and 4-ClAB consumption rate (i.e., rate of conversion to 4-ClAN) per atom of palladium exposed at a particle surface for the catalysts Ta1 to Ta2 and Ta4 to Ta5. Fig. 6 is a graph showing the relationship between Pd loading and Pd dispersion for the catalysts Ta1 to Ta2 and Ta4 to Ta5. Fig. 7 is a graph showing the relationship between Pd loading and Pd particle diameter for the catalysts Ta1 to Ta2 and Ta4 to Ta5.

Metal dispersion (PD dispersion here) in the hydrogenation catalyst can be calculated by a known method (a CO pulse method and TEM observation here).

The CO pulse method (CO adsorption measurement) assumes that metal is supported as a spherical body on a surface of a carrier, and that one CO atom adsorbs one PD atom exposed at the surface. Metal dispersion D (%) was calculated using the following formula (1).

$$\text{Metal dispersion D (\%)} = (n/N) * 100 \cdots (1)$$

where

n: mole number of adsorption gas per 1g of sample (mol/g)
N: mole number of metal per 1g of sample (mol/g)

Also, metal surface area per 1g of sample ($m^2/g$) can be calculated from the following formula (2).

$$S = n * 6.02 * 10^{23} * \sigma * 10^{-18} \cdots (2)$$

where

n: mole number (mol/g) of adsorption gas per 1g of sample
$\sigma$: cross-sectional area ($nm^2$/atom) of metal atom

Further, CO adsorption amount $V_{CO}$ (adsorption gas volume per 1g of sample at 0 °C and one atmosphere) can be calculated using the following formula (3).

$$V_{CO} = (V_{CO}_t/W) * (273/(273+t)) * (P/101.325) \cdots (3)$$

where

$V_{CO}_t/W$: adsorption gas volume ($cm^3$) at temperature t (°C) at the time of measurement
W: mass (g) of sample
P: pressure (kPa) at the time of measurement

Furthermore, a ratio of area to volume per 1g of metal is rearranged using surface area and density to develop the following formula (4), and radius r of the metal particle can be thus calculated.

$$r = 3C/(S * \rho * 108) \cdots (4)$$

where

C: metal content (wt. %)
S: metal surface area ($m^2/g$) per 1g of sample
$\rho$: metal density ($g/cm^3$)

Table 2 illustrates analysis results, obtained by the CO pulse method, of the catalysts Ta1 to Ta5.

[Table 2]

| | Pd loading (theoretical value) (wt%) | Pd loading (ICPmeasured value) (wt%) | CO adsorption amount ($cm^3/g$) | Surface metal atom number (quantity/g) | Average particle diameter (nm) | Pd dispersion (%) |
|---|---|---|---|---|---|---|
| Catalyst T a 1 | 5 | 4.34 | 1.1 | $3.0\times10^{19}$ | 9.0 | 12 |
| Catalyst T a 2 | 2 | 1.77 | 0.55 | $1.5\times10^{19}$ | 7.5 | 15 |
| Catalyst T a 3 | 0.4 | 0.37 | 0.35 | $0.93\times10^{19}$ | 2.5 | 45 |
| Catalyst T a 4 | 0.2 | 0.17 | 0.16 | $0.43\times10^{19}$ | 2.5 | 45 |
| Catalyst T a 5 | 0.05 | 0.05 | 0.029 | $0.078\times10^{19}$ | 3.9 | 29 |

Table 2 indicates Pd loading (wt. %), CO adsorption amount ($cm^3/g$), surface metal atom number (number/g), average particle diameter (nm), and PD dispersion (%) for each of the catalysts Ta1 to to Ta5. As Pd loading (wt. %) indicated are theoretical values prior to catalyst preparation and measured values, after catalyst preparation, by ICP analysis. The CO adsorption amount can be obtained from the above-described formula (3). The surface metal atom number (number/g) is the number of catalyst metal atoms exposed at a particle surface. The average particle diameter (nm) can be obtained by using 2r in the above-described formula (4). The PD dispersion can be obtained from the above-described formula (1).

Metal dispersion based on TEM can be also calculated using the above-described formula (1). In this case, an equivalent to n (mol/g) in the formula 2 was calculated from the relationship between S ($m^2/g$), $\sigma$ ($nm^2$), and n (mol/g). Besides, S ($m^2/g$) can be calculated using the above-described formula (4) from the relationship between radius r (m) of the catalyst metal, which can be obtained from average particle diameter as measured by TEM, and the metal content C (wt. %) of the sample.

With equivalent metal loadings, the hydrogenation catalyst according to the present embodiment has lower metal dispersion based on the CO pulse method in some cases than that based on TEM observation. Thus, catalyst performance is evaluated based on CO adsorption inhibition rate of the hydrogenation catalyst determined by the following formula (5).

[Math. 2]

$$\text{CO adsorption inhibition rate (\%)} = \left(1 - \frac{D_{CO}}{D_{TEM}}\right) \times 100 \quad \cdots (5)$$

where

$D_{CO}$: metal dispersion based on the CO pulse method
$D_{TEM}$: metal dispersion based on TEM (transmission electron microscope) observation.

As illustrated in Fig. 5, it can be seen that consumption rate of 4-ClAB increases as the CO adsorption inhibition rate increases (i.e., in the order of Ta1, Ta2, Ta4, and Ta5).

Arranged in Table 3 is data on the CO adsorption inhibition rate of the catalysts Ta1, Ta2, Ta4, and Ta5 and the like as shown in Fig. 5.

[Table 3]

| | Pd loading (theoretical value) (wt%) | Pd loading (ICPmeasured value) (wt%) | Average particle diameter (nm) | CO adsorption inhibition rate (%) |
|---|---|---|---|---|
| Catalyst T a 1 | 5 | 4.34 | 9.0 | 0 |
| Catalyst T a 2 | 2 | 1.77 | 7.5 | 6 |
| Catalyst T a 4 | 0.2 | 0.17 | 2.5 | 44 |
| Catalyst T a 5 | 0.05 | 0.05 | 3.9 | 61 |

When the results as shown above in Fig. 2 are taken into consideration, the CO adsorption inhibition rate is preferably 61% or less to avoid a decrease in catalytic activity caused by a decrease in palladium to be involved in the hydrogenation reaction.

As illustrated in Fig. 6, the metal dispersion (%) and the CO adsorption inhibition rate each tend to increase as the Pd loading (wt. %) decreases. As illustrated in Fig. 7, the average palladium particle diameter (nm) tends to decrease as the Pd loading (wt. %) decreases.

Figs. 6 and 7 respectively indicate metal dispersion based on TEM observation and average palladium particle diameter of a catalyst that was prepared by a method similar to that used for the catalyst Ta2 with the exception that calcination temperature was changed to 400 °C. There results confirm that the metal dispersion and the average palladium particle diameter are changed by a change in calcination temperature.

Fig. 8 is a graph showing the relationship between the conversion of 4-ClAB as a liquid raw material and the selectivity of 4-ClAN in a hydrogenation reaction for the catalysts Ta4, Tb1, S1, A1, and H1 and the Pd/C catalyst (catalyst for comparison).

Fig. 8 confirms that the selectivity of 4-ClAN exhibited by each of the catalysts Ta4, Tb1, S1, A1, and H1 is higher than that exhibited by the Pd/C catalyst for the conversion of 4-ClAB of a similar value.

Fig. 9A is a graph showing the relationship between the conversion of 4-ClAB and the selectivity of 4-ClAN in the hydrogenation reaction, and Fig. 9B is a graph showing the relationship between the conversion of 4-ClAB and the selectivity of a by-product, each for the catalysts Ta2 and Ta4 and the Pd/C catalyst (catalyst for comparison). The catalysts Ta2 and Ta4 have average palladium particle diameters of 7.0 nm and 1.4 nm, respectively.

As illustrated in Fig. 9A, the smaller the average palladium particle diameter thereof, the higher selectivity of 4-ClAN the Pd-supported titania catalysts exhibit, compared with the Pd/C catalyst. As illustrated in Fig. 9B, in contrast, the smaller the average palladium particle diameter thereof, the lower selectivity of the by-product the Pd-supported titania catalysts exhibit, compared with the Pd/C catalyst.

Fig.10A is a graph showing the relationship between the conversion of 4-ClAB and the selectivity of 4-ClAN in the hydrogenation reaction, and Fig. 10B is a graph showing the relationship between the conversion of 4-ClAB and the selectivity of a by-product, each for the catalysts A1 and A2 and the Pd/C catalyst (catalyst for comparison). The catalyst A1 has an average palladium particle diameter of 3.1 nm.

When the average palladium particle diameter is 3.1 nm, as illustrated in Fig. 10A, the Pd-supported alumina catalyst exhibits higher selectivity of 4-ClAN than the Pd/C catalyst. When the average palladium particle diameter is 3.1 nm, in contrast, the Pd-supported alumina catalyst exhibits lower selectivity of the by-product than the Pd/C catalyst, as illustrated in Fig. 10B.

Fig.11A is a graph showing the relationship between the conversion of 4-ClAB and the selectivity of 4-ClAN in the hydrogenation reaction, and Fig. 10B is a graph showing the relationship between the conversion of 4-ClAB and the selectivity of a by-product, each for the catalysts H1 and H2 and the Pd/C catalyst (catalyst for comparison). The catalyst H1 has an average palladium particle diameter of 2.7 nm.

When the average palladium particle diameter is 2.5 nm, as illustrated in Fig. 11A, the Pd-supported titania-coating alumina catalyst exhibits higher selectivity of 4-ClAN than the Pd/C catalyst. When the average palladium particle diameter is 2.5 nm, in contrast, the Pd-supported titania-coating alumina catalyst exhibits lower selectivity of the by-product than the Pd/C catalyst, as illustrated in Fig. 11B.

As with the above-described catalysts Ta1, Ta2, Ta4, and Ta5 (refer to Table 3), data on the CO adsorption inhibition rate of the catalysts A1 and H1 and the like is arranged in Table 4.

[Table 4]

| | Pd loading (theoretical value) (wt%) | Pd loading (ICPmeasured value) (wt%) | Average particle diameter (nm) | CO adsorption inhibition rate (%) |
|---|---|---|---|---|
| Catalyst A 1 | 0.2 | 0.188 | 4 | 22.6 |
| Catalyst H 1 | 0.2 | 0.179 | 2.9 | 6.1 |

Fig.12A is a graph showing the relationship between the conversion of 4-ClAB and the selectivity of 4-ClAN in the hydrogenation reaction, and Fig. 12B is a graph showing the relationship between the conversion of 4-ClAB and the selectivity of a by-product, each for the catalysts Tc1 and Tc2 and the Pd/C catalyst (catalyst for comparison). The catalysts Tc1 and Tc2 have average palladium particle diameters of 3.2 nm and 7.2 nm, respectively.

As illustrated in Fig. 12A, the smaller the average palladium particle diameter thereof, the higher selectivity of 4-ClAN the Pd-supported titania catalysts exhibit, compared with the Pd/C catalyst, even when titania for a catalytic carrier is changed to a different product than the catalysts Ta2 and Ta4. As illustrated in Fig. 12B, in contrast, the smaller the average

palladium particle diameter thereof, the lower selectivity of the by-product the Pd-supported titania catalysts exhibit, compared with the Pd/C catalyst, even when titania for a catalytic carrier is changed to a different product than the catalysts Ta2 and Ta4.

Fig.13A is a graph showing the relationship between the conversion of 4-ClAB and the selectivity of 4-ClAN in the hydrogenation reaction, and Fig. 13B is a graph showing the relationship between the conversion of 4-ClAB and the selectivity of a by-product, each for the catalysts Tb1 and Tb2 and the Pd/C catalyst (catalyst for comparison). The catalysts Tb1 and Tb2 have average palladium particle diameters of 5.8 nm and 17.9 nm, respectively.

As illustrated in Fig. 13A, the Pd-supported titania catalysts exhibit higher selectivity of 4-ClAN than the Pd/C catalyst, even when titania for a catalytic carrier is changed to a different crystal type (100% rutile) than the catalysts Ta2 and Ta4. However, an improvement in selectivity could not be seen even when the average palladium particle diameter is decreased. As illustrated in Fig. 13B, in contrast, the Pd-supported titania catalysts exhibit lower selectivity of the by-product than the Pd/C catalyst, even when titania for a catalytic carrier is changed to a different crystal type (100% rutile) than the catalysts Ta2 and Ta4. However, a reduction in selectivity could not be seen even when the average palladium particle diameter is decreased.

Thus, the Pd-supported titania catalyst and the Pd-supported alumina catalyst according to the present embodiment inhibits, in hydrogenation of an aromatic halonitro compound, a reduction in selectivity of a halogenated aromatic amine as a target product caused by proceeding of a halogen elimination reaction (excessive reaction). To obtain similar effects, a Pt-supported titania catalyst as disclosed in a reference set forth below requires activation treatment such as of hydrogen reduction at least at or above 250 °C. However, the Pd-supported titania catalyst and the Pd-supported alumina catalyst according to the present embodiment improves the selectivity of halogenated aromatic amine as the target product even without such activation treatment. Therefore, use of the Pd-supported titania catalyst and the Pd-supported alumina catalyst according to the present embodiment allows hydrogenation of an aromatic halonitro compound, while simplifying a production process of a hydrogenated organic compound.

Reference cited: Angew. Chem. Int. Ed., 2020, 59, 11824-11829, "Strong Interactions between Pt Single Atoms and TiO2"

Fig.14A is a graph showing the relationship between the conversion of 4-ClAB and the selectivity of 4-ClAN in the hydrogenation reaction, and Fig. 14B is a graph showing the relationship between the conversion of 4-ClAB and the selectivity of a by-product, each for the catalysts S1 and S2 and the Pd/C catalyst (catalyst for comparison). The catalysts S1 and S2 have average palladium particle diameters of 3.5 nm and 5.5 nm, respectively.

As illustrated Fig. 14A, the selectivity of 4-ClAN exhibited by Pd-supported silica catalysts is similar to that by the Pd/C catalyst regardless of variations in the average palladium particle diameter. As illustrated Fig. 14B, the selectivity of the by-product exhibited by the Pd-supported silica catalysts is similar to that by the Pd/C catalyst regardless of variations in the average palladium particle diameter.

Fig. 15 is a graph showing the relationship between the conversion of 4-ClAB as a liquid raw material and the selectivity of a nitroso compound (4-chloronitrosobenzene here) in the hydrogenation reaction for the catalyst S2, the catalyst Td1, the catalyst A2, the catalyst H2, and the catalyst Pd/C (catalyst for comparison). Fig. 16 is a graph showing the relationship between the conversion of 4-ClAB and the selectivity of 4-ClAN in the hydrogenation reaction for the same catalysts as those in Fig. 15.

Fig. 15 confirms that the selectivity of the nitroso compound exhibited by each of the catalyst S2, the catalyst Td1, the catalyst A2, and the catalyst H2 is lower than that exhibited by the Pd/C catalyst for the conversion of 4-ClAB of a similar value. Particularly the catalyst Td1 including titania produces little nitroso compound.

Fig. 16 confirms that the selectivity of 4-ClAB exhibited by the catalyst Td1 is higher than that exhibited by the Pd/C catalyst for the conversion of 4-ClAB of a similar value. Also, Fig. 16 confirms that the selectivity of 4-ClAN exhibited by the catalyst S1 is slightly lower than that exhibited by the Pd/C catalyst for the conversion of 4-ClAB of some values (approximately 80%) but is basically equal to or higher than the same.

Figs. 17 and 18 are graphs showing effects of carrier calcination temperatures (500 °C, 600 °C, 700 °C, 800 °C, 900 °C, and 950 °C) on the action (reaction characteristics) of the catalyst using the carrier including titania. Fig. 17 shows the relationship between the conversion of 4-ClAB as a liquid raw material and the selectivity of a nitroso compound (4-chloronitrosobenzene here) in the hydrogenation reaction for the catalyst Ta2 with each of the foregoing carrier calcination temperatures.

Fig. 18 shows the relationship between the conversion of 4-ClAB as the liquid raw material and the selectivity of 4-ClAN in the hydrogenation reaction for the catalyst Ta2 with the same carrier calcination temperatures as in Fig. 17.

As illustrated in Fig. 17, the catalyst Ta2 with the carrier calcination temperatures 500 °C, 600 °C, and 700 °C produces little nitroso compound (the selectivity of the nitroso compound is approximately zero). In contrast, production of the nitroso compound is seen for the catalyst Ta2 with higher carrier calcination temperatures (800 °C and above). Particularly the catalyst Ta2 with the carrier calcination temperatures 900 °C and 950 °C (900 °C and above) causes a significant increase in production of the nitroso compound, and thus exhibits the selectivity of the nitroso compound equal to or higher than that exhibited by the Pd/C catalyst.

Fig. 18 confirms that the catalyst Ta2 with any of the carrier calcination temperatures tends to exhibit higher

selectivity of 4-ClAN than the Pd/C catalyst.

Fig. 19 is a graph showing X-ray diffraction patterns based on an X-ray diffraction method (XRD) with regard to the carrier of the catalyst Ta2 with the same carrier calcination temperatures as in Figs. 17 and 18. As an X-ray diffraction device, a fully automatic multi-purpose horizontal X-ray analyzer (Smart Lab X-RAY DIFFRACTOMETER) manufactured by Rigaku Corporation was used. Also, quantitative analysis in the X-ray diffraction method was conducted by an RIR (Reference Intensity Ratio) method.

Detected in the diffraction pattern of the X-ray shown in Fig. 6A was a peak (which appeared in the vicinity of a diffraction angle $2\theta$ = 25.3° in a case where a CuK$\alpha$ ray was used as an X-ray source) corresponding to a {101} surface of the anatase crystal phase that indicates the presence of titania. With regard to the carrier calcination temperatures (800 °C, 900 °C, and 950 °C) where the nitroso compound was produced, by contrast, a peak (which appeared in the vicinity of a diffraction angle $2\theta$ = 27.4° in a case where a CuK$\alpha$ ray was used as an X-ray source) corresponding to a {110} surface of a rutile crystal phase was detected in addition to the anatase peak. With regard to the carrier calcination temperatures (900 °C and 950 °C) where a significant increase was seen in production of the nitroso compound, the anatase peak decreased, whereas the rutile peak intensity was significantly high. This indicates a correlation between the percentage of the rutile crystal phase and the production behavior of the nitroso compound. In other words, the catalyst including titania inhibits the production of the nitroso compound by an increase in percentage of the anatase crystal phase.

Table 5 shows the percentage of each of the crystal phases (anatase and rutile) calculated based on the RIR method from X-ray diffraction data on each of the carrier calcination temperatures (500 °C, 600 °C, 700 °C, 800 °C, 900 °C, and 950 °C) of the catalyst Ta2.

[Table 5]

| Number | Calcination temperature | Percentage of crystal phase | |
|---|---|---|---|
| | | Anatase | Rutile |
| 1 | 950°C | 7.5 | 92.5 |
| 2 | 900°C | 38 | 62 |
| 3 | 800°C | 96.4 | 3.6 |
| 4 | 700°C | 100 | 0 |
| 5 | 600°C | 100 | 0 |
| 6 | 500°C | 100 | 0 |

When the selectivity of 4-ClAN and the selectivity of the nitroso compound, as shown in each of Figs. 17 and 18, and the percentage of the anatase crystal phase, as shown in Table 5, are taken into consideration with regard to the catalyst Ta2, it can be seen that in a case where the carrier including titania has 38% or more anatase crystal phase, the yield of 4-ClAN (target product) is improved, while the production of the nitroso compound is inhibited, as compared with the Pd/C catalyst. The calcination temperatures of the carrier including titania at 900 °C or below allow the percentage of the anatase crystal phase to be 38% or more.

Figs. 20 and 21 are graphs showing effects of average pore diameters of carriers including titania on the action of catalysts using the carriers. Fig. 20 shows the relationship between the conversion of 4-ClNB as a liquid raw material and the selectivity of the nitroso compound (4-chloronitrosobenzene here) in the hydrogenation reaction for the catalyst Td1, the catalyst Ta2, the catalyst Te1, and the Pd/C catalyst (catalyst for comparison). Fig. 21 shows the relationship between the conversion of 4-ClNB as the liquid raw material and the selectivity of 4-ClAN in the hydrogenation reaction for the same catalysts as in Fig. 20.

Table 6 indicates average pore diameter of the carrier used in each of the catalyst Td1, the catalyst Ta2, and the catalyst Te1. Each average pore diameter was measured based on mercury porosimetry. As a pore distribution measuring device used was "Micromeritics Automatic Mercury Porosimeter Autopore IV 9500" manufactured by Shimadzu Corporation.

[Table 6]

| Catalyst | Average pore diameter (4V/A) (nm) | Substrate length (nm) | (Average pore diameter/ Substrate length) Ratio (-) |
|---|---|---|---|
| C($TiO_2$) | 7.3 | 0.65 | 11.2 |
| D($TiO_2$) | 18.1 | 0.65 | 27.8 |

(continued)

| Catalyst | Average pore diameter (4V/A) (nm) | Substrate length (nm) | (Average pore diameter/ Substrate length) Ratio (-) |
|---|---|---|---|
| E($TiO_2$) | 50.7 | 0.65 | 78 |

Fig. 20 confirms that the selectivity of the nitroso compound exhibited by each of the catalyst Td1, the catalyst Ta2, and the catalyst Te1 is lower than that exhibited by the Pd/C catalyst for the conversion of 4-ClAB of a similar value. Particularly the catalyst Td1 and the catalyst Ta2 with smaller average pore diameters produce little nitroso compound.

Fig. 21 confirms that the selectivity of 4-ClAN exhibited by each of the catalyst Td1, the catalyst Ta2, and the catalyst Te1 tends to be higher than that exhibited by the Pd/C catalyst for the conversion of 4-ClAB of a similar value.

When the selectivity of 4-ClAN and the selectivity of the nitroso compound, as shown in each of Figs. 20 and 21, and the average pore diameters of the carrier as shown in Table 2 are taken into consideration with regard to the catalyst Td1, the catalyst Ta2, and the catalyst Te1 using the carrier including titania, it can be seen that in a case where average pore diameter of the carrier including titania is approximately 50 nm or less, the yield of 4-ClAN (target product) is improved, while the production of the nitroso compound is inhibited, as compared with the Pd/C catalyst. It can also be seen that an average pore diameter of approximately 18 nm or less as in the catalysts Td1 and D is more preferable from the viewpoint of inhibiting the production of the nitroso compound and improving the yield of 4-ClAN (target product). Further, it can be seen that an average pore diameter of approximately 7 nm or less as in the catalyst Td1 is still more preferable from the viewpoint of inhibiting the production of the nitroso compound and improving the yield of 4-ClAN (target product). Further, as shown in Table 2, the average pore diameter of the carrier including titania is preferably 78 times or less as long as longitudinal molecular length (substrate length) of 4-ClNB. This stably improves the yield of 4-ClAN as the target product while effectively inhibiting the production of the nitroso compound. Furthermore, the average pore diameter of the carrier including titania is more preferably 28 times or less, and still more preferably 11 times or less, as long as the substrate length.

As described above, the hydrogenation catalyst according to the embodiments has advantageous effects, particularly when used in hydrogenation of aromatic halonitro compounds, over conventional catalysts used in the hydrogenation of aromatic halonitro compounds. In hydrogenation of an aromatic halonitro compound, more specifically, the hydrogenation catalyst improves the yield of a halogenated aromatic amine (hydrogenated organic compound) as a target product without the need for a dehalogenation inhibitor while inhibiting the production of a nitroso compound.

The present invention has been described above based on specific embodiments, but these embodiments are merely examples, and the present invention is not limited by these embodiments. Not all the components of the hydrogenation catalyst and the production method of the hydrogenated organic compound using the hydrogenation catalyst shown in the above-mentioned embodiments are essential, and at least those skilled in the art can select the components as appropriate within the scope of the present invention.

[Reference Signs List]

1: flow-type organic synthesis system
2: flow-type organic synthesis system
3: catalyst layer
4: electric furnace
11: liquid raw material tank
12: liquid raw material pump
13: preheater/precooler
21: heat exchanger
25: main drum
41: product recovery drum

## Claims

**1.** A hydrogenation catalyst that is used in hydrogenation of an aromatic halonitro compound, comprising:

a carrier including at least one of titania and alumina; and

palladium supported on the carrier,

wherein the palladium has an average particle diameter of 9.7 nm or less.

2. The hydrogenation catalyst according to claim 1, having a CO adsorption inhibition rate, indicated by the following formula, of 61% or less:

[Math. 3]

$$\text{CO adsorption inhibition rate (\%)} = \left(1 - \frac{D_{CO}}{D_{TEM}}\right) \times 100$$

where $D_{CO}$ is metal dispersion based on a CO pulse method, and $D_{TEM}$ is metal dispersion based on TEM (transmission electron microscope) observation.

3. The hydrogenation catalyst according to claim 1 or claim 2, wherein:

the carrier is a composite carrier consisting of alumina and titania; and
the composite carrier includes a structure where a base material consisting of alumina is coated with titania.

4. The hydrogenation catalyst according to claim 1 or claim 2, wherein the carrier including titania includes titania with 38% or more anatase crystal structure.

5. The hydrogenation catalyst according to claim 4, wherein the carrier including titania does not include a rutile crystal structure.

6. The hydrogenation catalyst according to claim 1 or claim 2, wherein the carrier including titania has an average pore diameter that is 78 times or less as long as longitudinal molecular length of the aromatic halonitro compound.

7. The hydrogenation catalyst according to claim 1 or claim 2, wherein the carrier has an average pore diameter of approximately 50 nm or less.

8. A flow-type organic synthesis system that is configured to cause a gas-liquid-solid three-phase reaction by using:

a fixed catalyst including the hydrogenation catalyst according to any one of claims 1 to 7;
hydrogen as a gas raw material; and
an aromatic halonitro compound as a liquid raw material.

9. A method for producing a hydrogenated organic compound, comprising performing hydrogenation of the aromatic halonitro compound by use of the hydrogenation catalyst according to any one of claims 1 to 7.

10. A method for producing a hydrogenated organic compound according to claim 9, wherein the hydrogenation of the aromatic halonitro is performed by use of the aromatic halonitro compound without being activated at or above 250 °C.

Fig.1

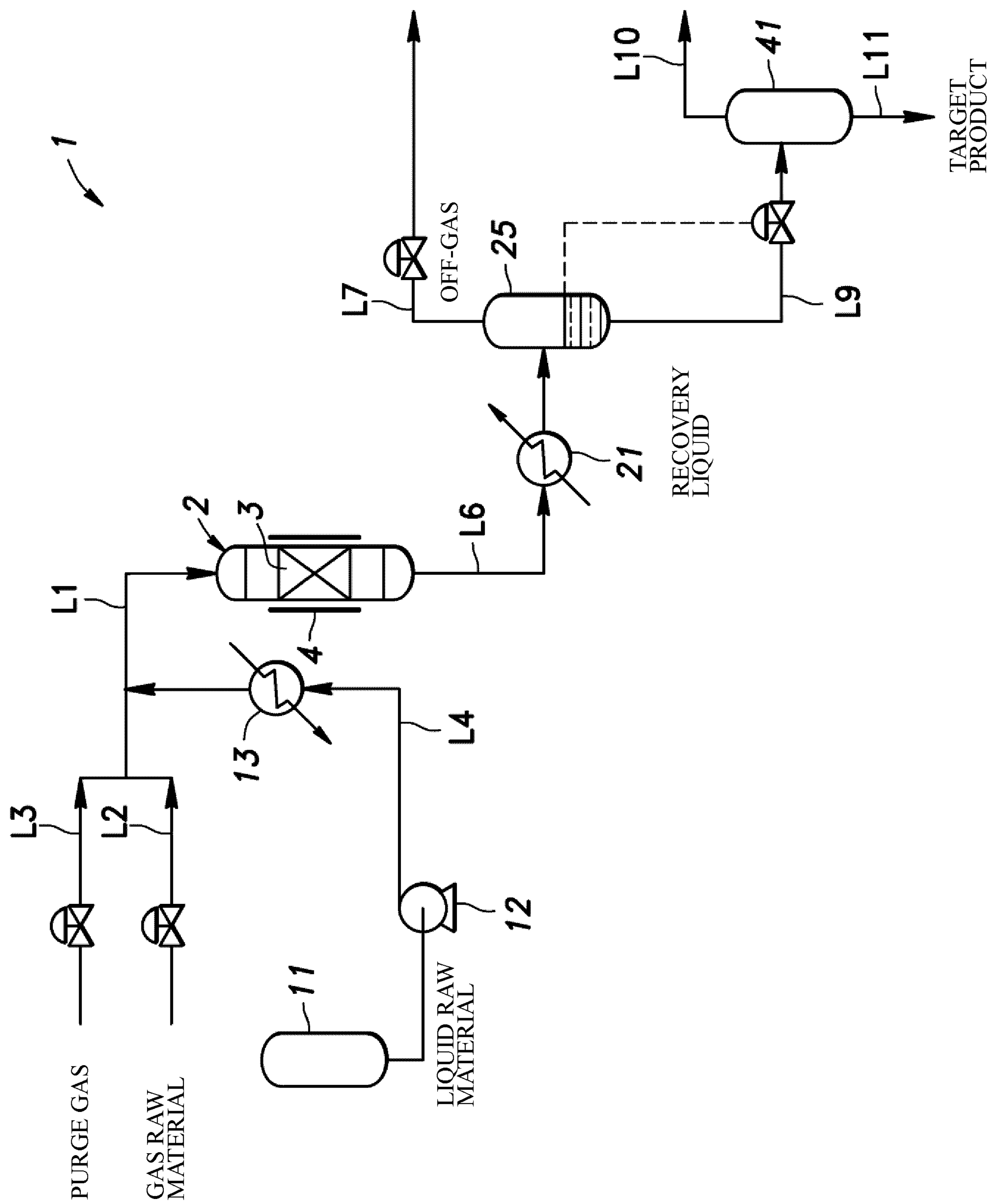
1
PURGE GAS
GAS RAW MATERIAL
L3
L2
L1
L4
11
LIQUID RAW MATERIAL
12
13
4
2
3
L6
21
RECOVERY LIQUID
25
L7
OFF-GAS
L9
41
L10
L11
TARGET PRODUCT

Fig.2

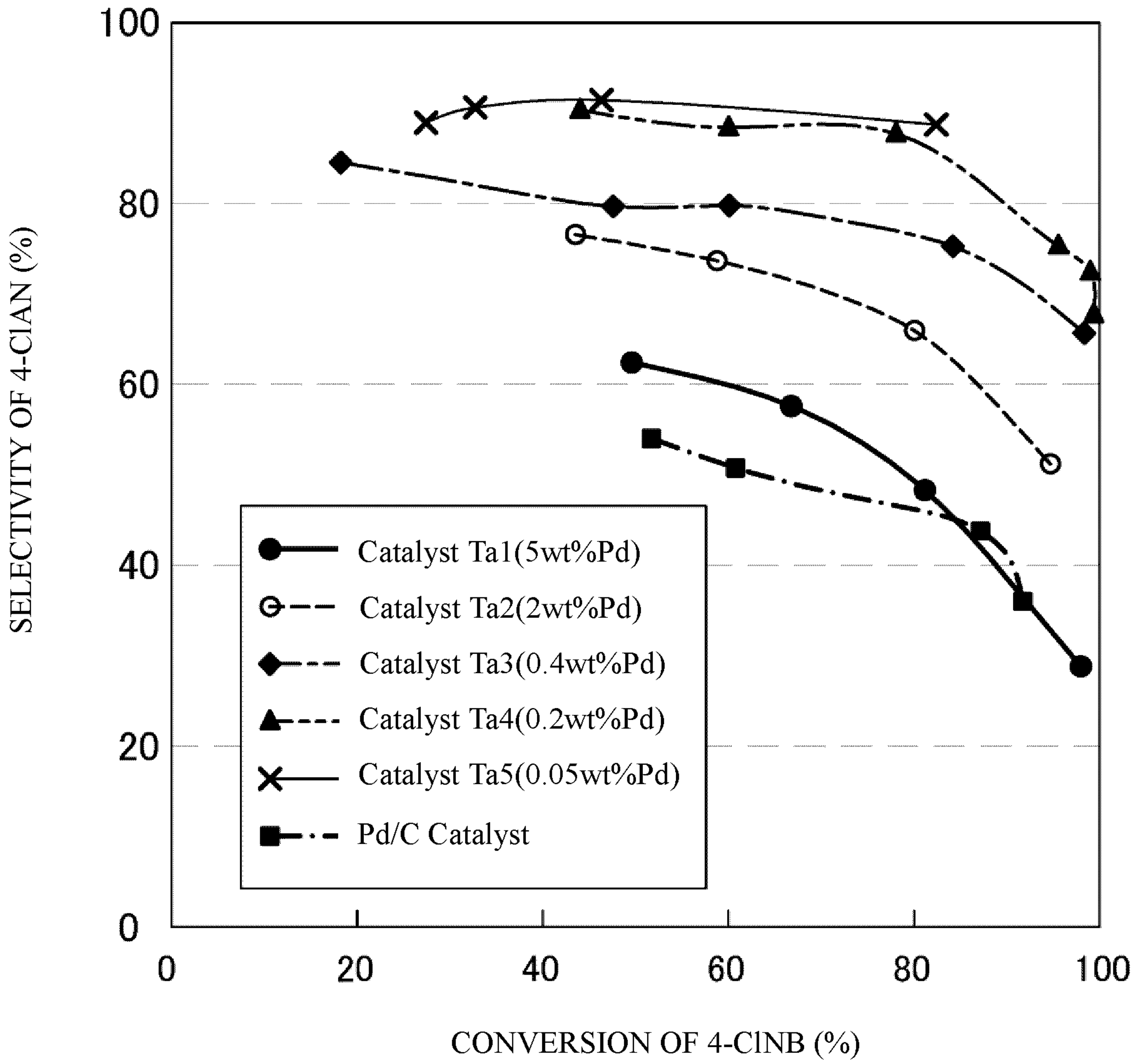
100
80
60
40
20
0
0
20
40
60
80
100
SELECTIVITY OF 4-ClAN (%)
CONVERSION OF 4-ClNB (%)
Catalyst Ta1(5wt%Pd)
Catalyst Ta2(2wt%Pd)
Catalyst Ta3(0.4wt%Pd)
Catalyst Ta4(0.2wt%Pd)
Catalyst Ta5(0.05wt%Pd)
Pd/C Catalyst

Fig.3A

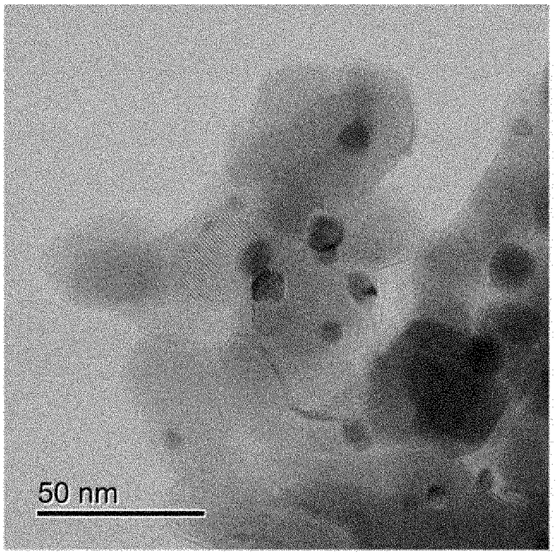


Catalyst Ta1

5wt% Pd, Pd particle diameter 9.7nm

Fig.3B

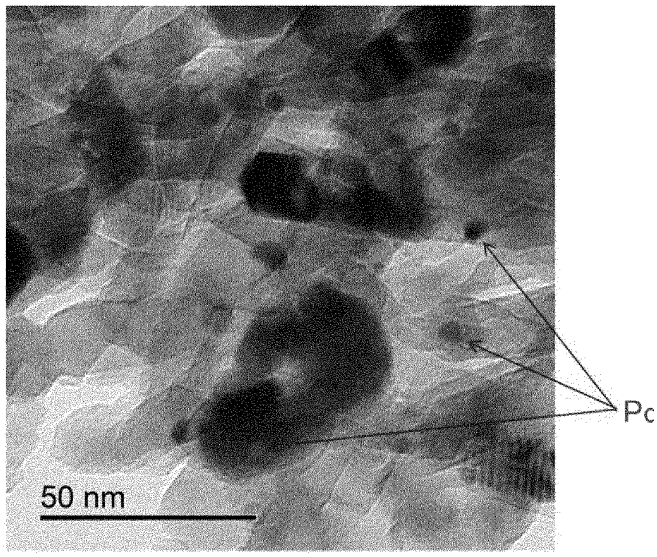


Catalyst Ta2

2wt% Pd, Pd particle diameter 7.0nm

Fig.3C

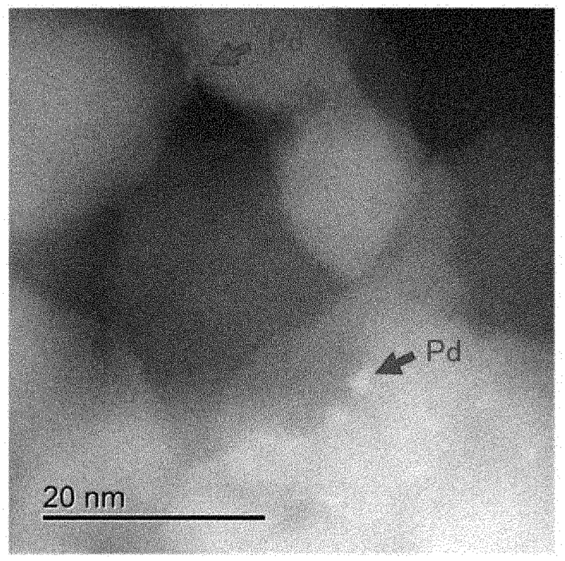


Catalyst Ta4

0.2wt% Pd, Pd particle diameter 1.4nm

Fig.3D

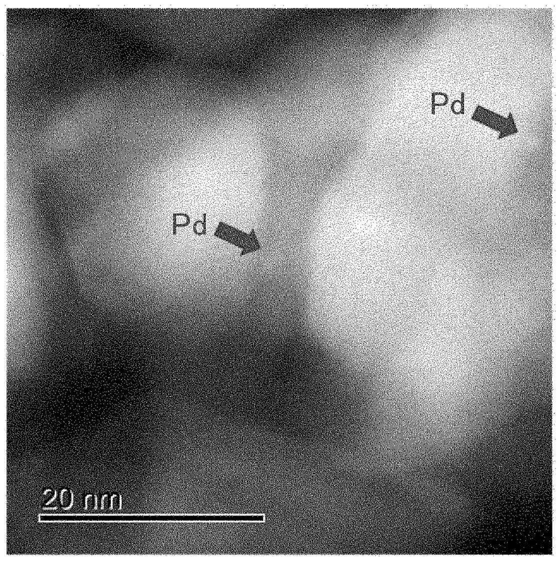


Catalyst Ta5

0.05wt% Pd, Pd particle diameter 1.5nm

Fig.4

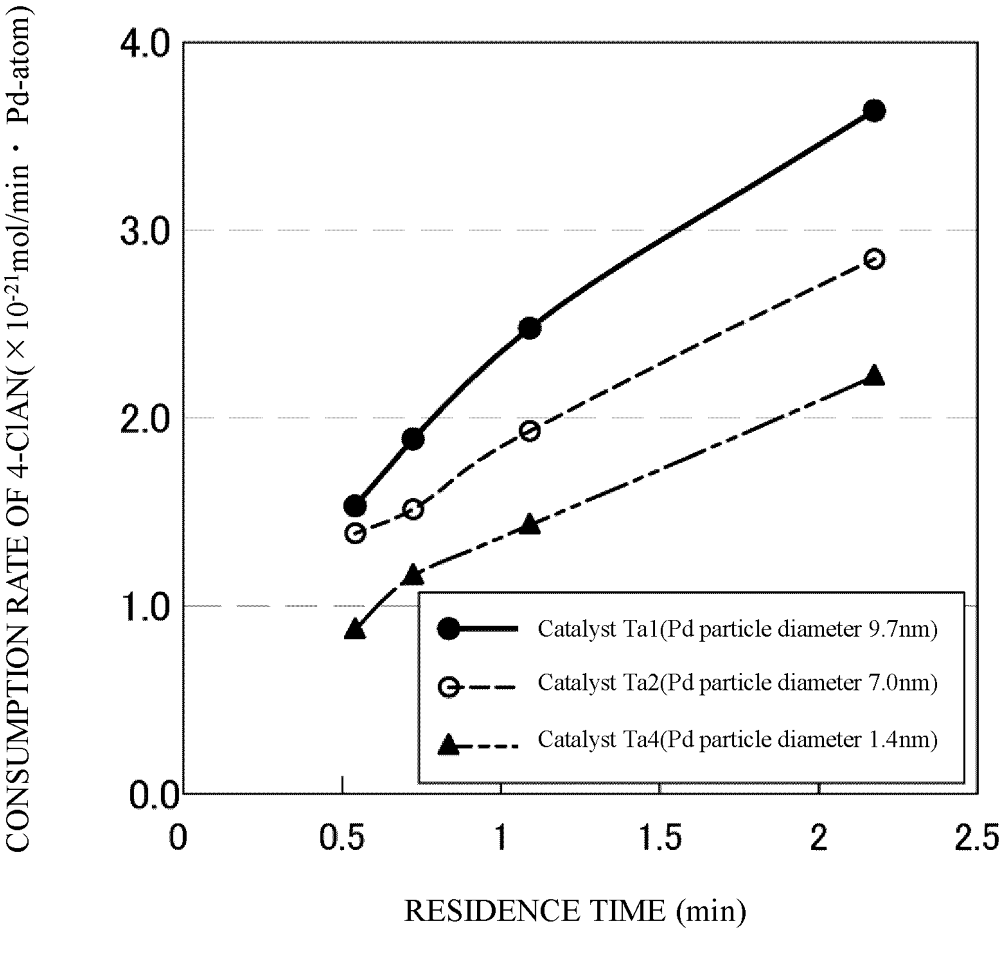
CONSUMPTION RATE OF 4-ClAN(×10-21mol/min・Pd-atom)
4.0
3.0
2.0
1.0
0.0
0
0.5
1
1.5
2
2.5
RESIDENCE TIME (min)
Catalyst Ta1(Pd particle diameter 9.7nm)
Catalyst Ta2(Pd particle diameter 7.0nm)
Catalyst Ta4(Pd particle diameter 1.4nm)

Fig.5

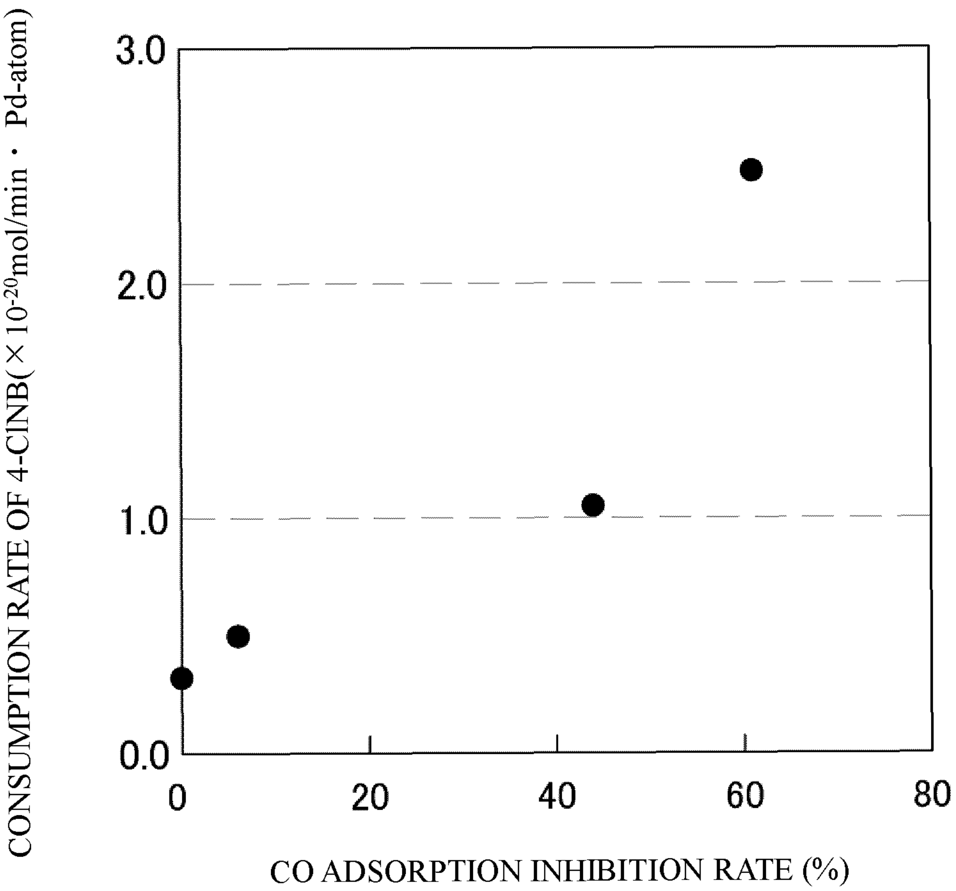
CONSUMPTION RATE OF 4-ClNB(×10-20mol/min・Pd-atom)
3.0
2.0
1.0
0.0
0
20
40
60
80
CO ADSORPTION INHIBITION RATE (%)

Fig.6

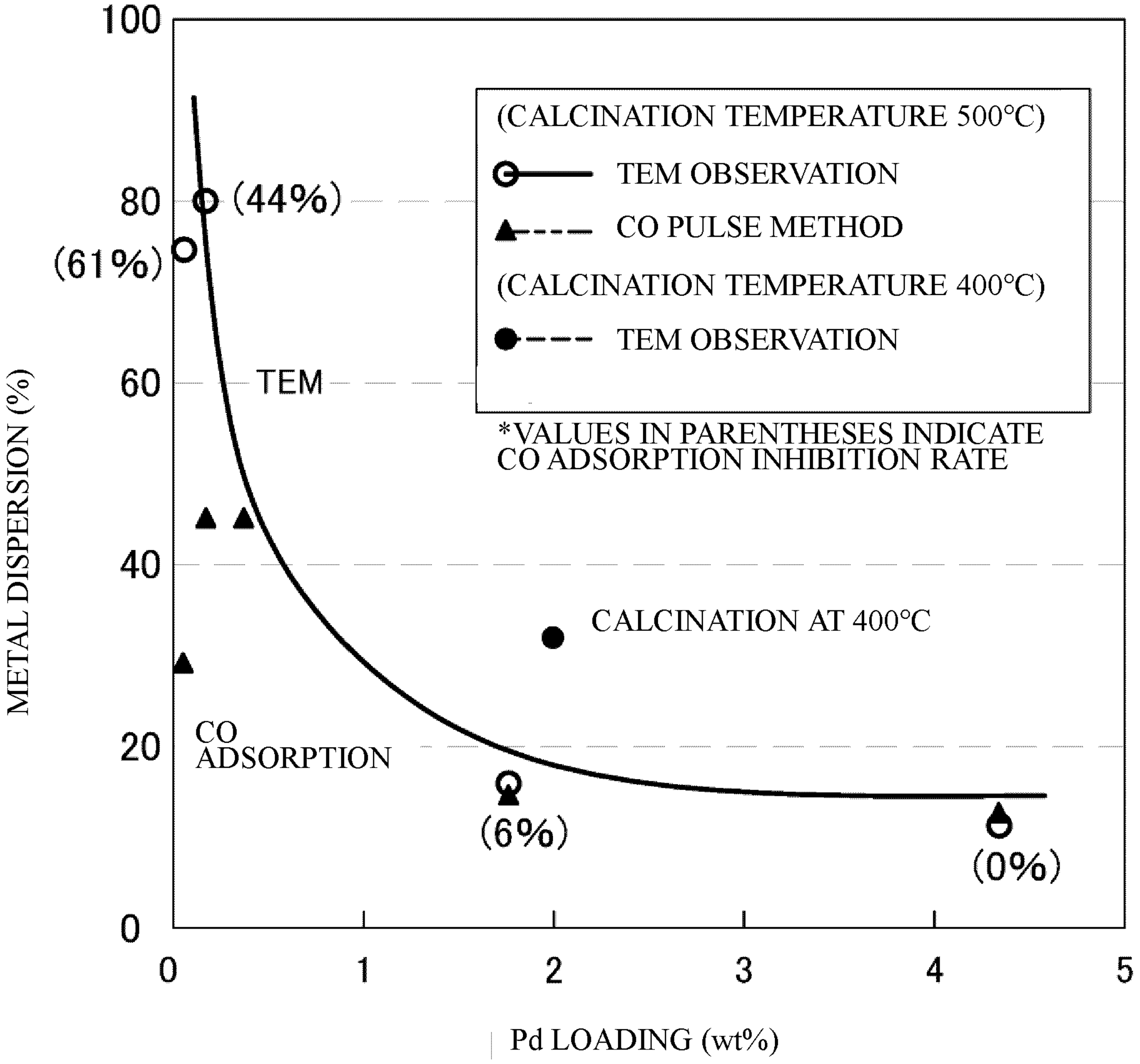
100
80
60
40
20
0
0
1
2
3
4
5
METAL DISPERSION (%)
Pd LOADING (wt%)
(CALCINATION TEMPERATURE 500°C)
TEM OBSERVATION
CO PULSE METHOD
(CALCINATION TEMPERATURE 400°C)
TEM OBSERVATION
*VALUES IN PARENTHESES INDICATE CO ADSORPTION INHIBITION RATE
(44%)
(61%)
TEM
CALCINATION AT 400°C
CO ADSORPTION
(6%)
(0%)

Fig.7

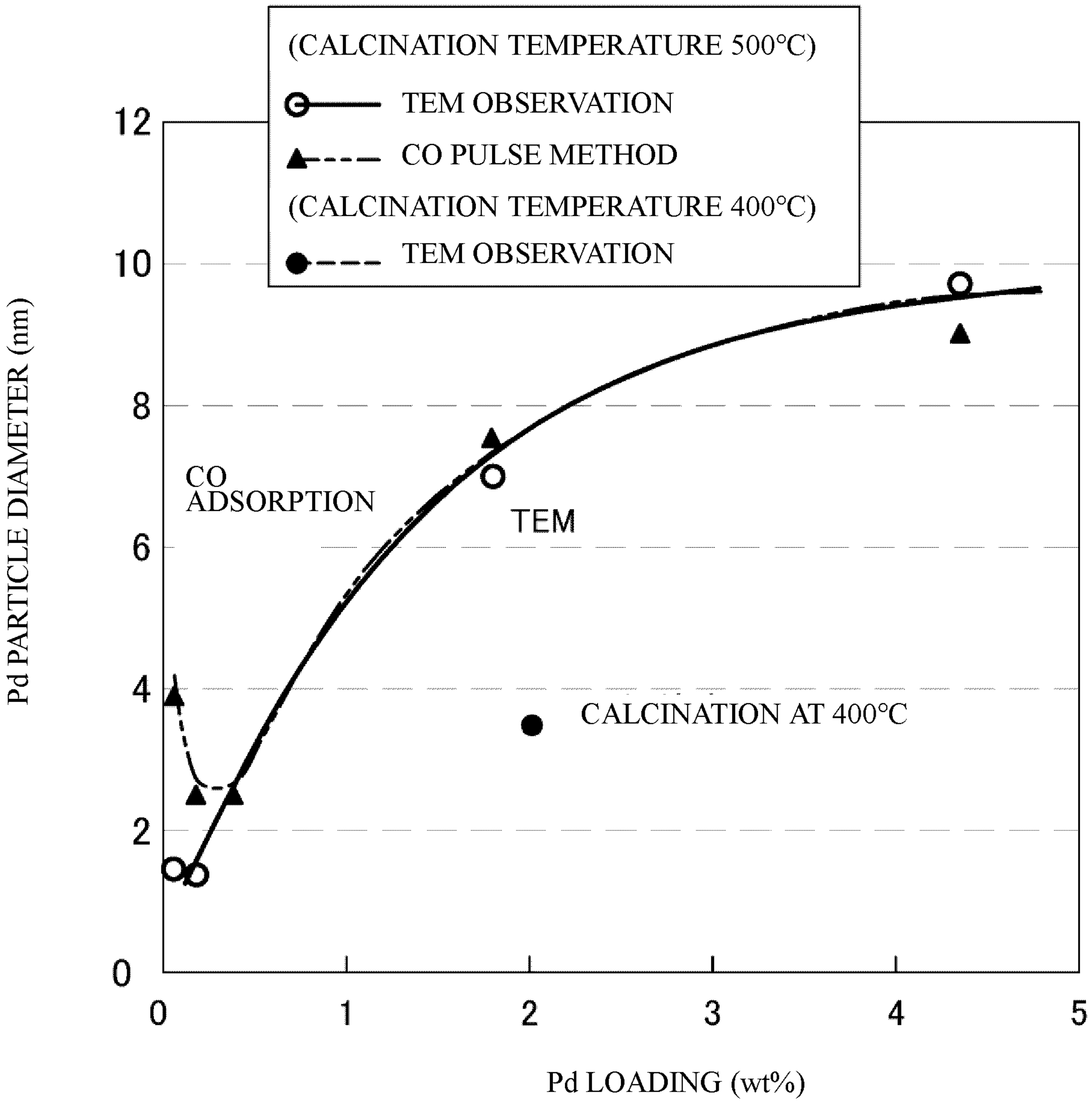
(CALCINATION TEMPERATURE 500°C)
TEM OBSERVATION
CO PULSE METHOD
(CALCINATION TEMPERATURE 400°C)
TEM OBSERVATION
12
10
8
6
4
2
0
Pd PARTICLE DIAMETER (nm)
CO
ADSORPTION
TEM
CALCINATION AT 400°C
0
1
2
3
4
5
Pd LOADING (wt%)

Fig.8

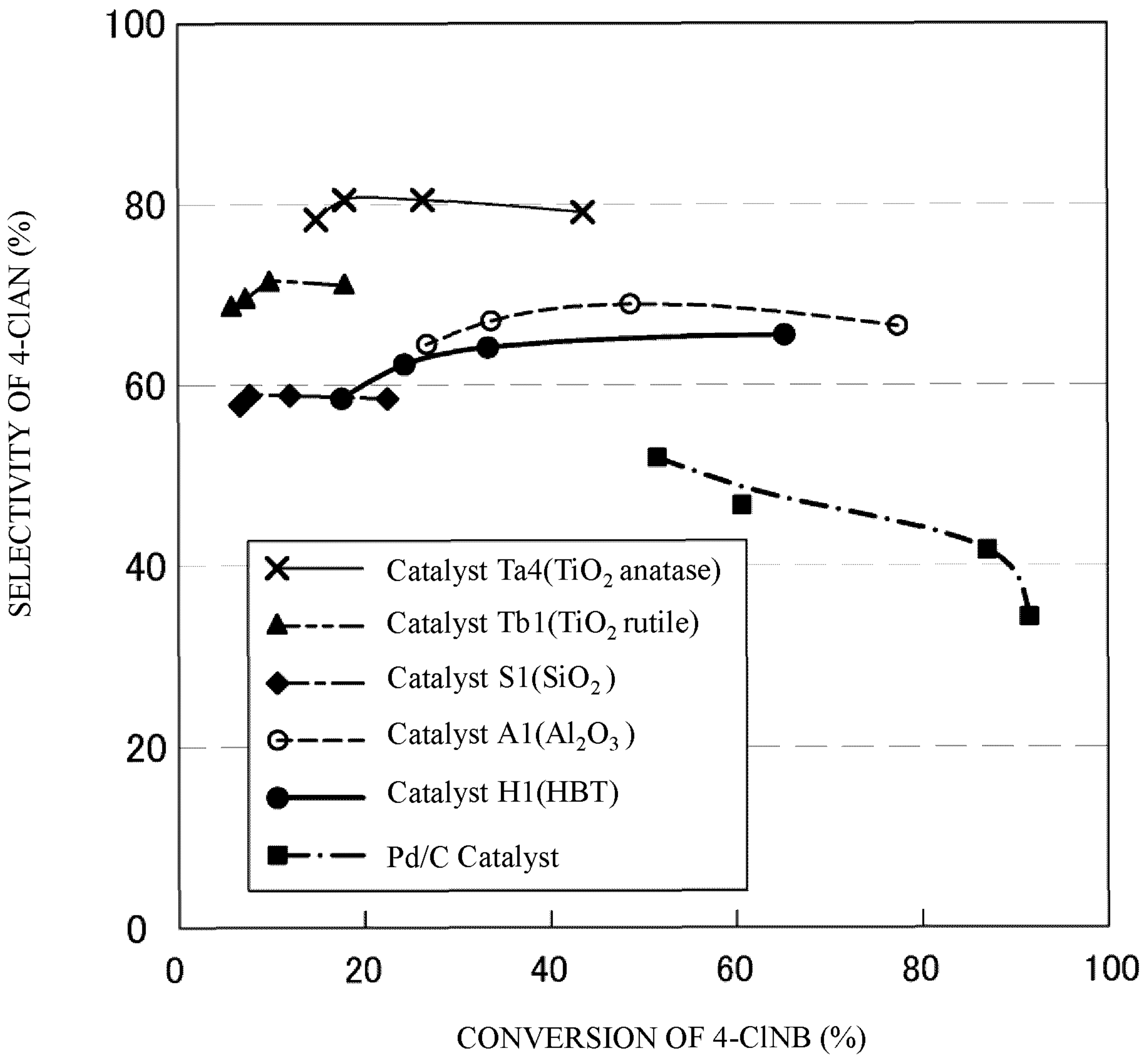
100
80
60
40
20
0
SELECTIVITY OF 4-ClAN (%)
0
20
40
60
80
100
CONVERSION OF 4-ClNB (%)
Catalyst Ta4(TiO2 anatase)
Catalyst Tb1(TiO2 rutile)
Catalyst S1(SiO2)
Catalyst A1(Al2O3)
Catalyst H1(HBT)
Pd/C Catalyst

Fig.9A

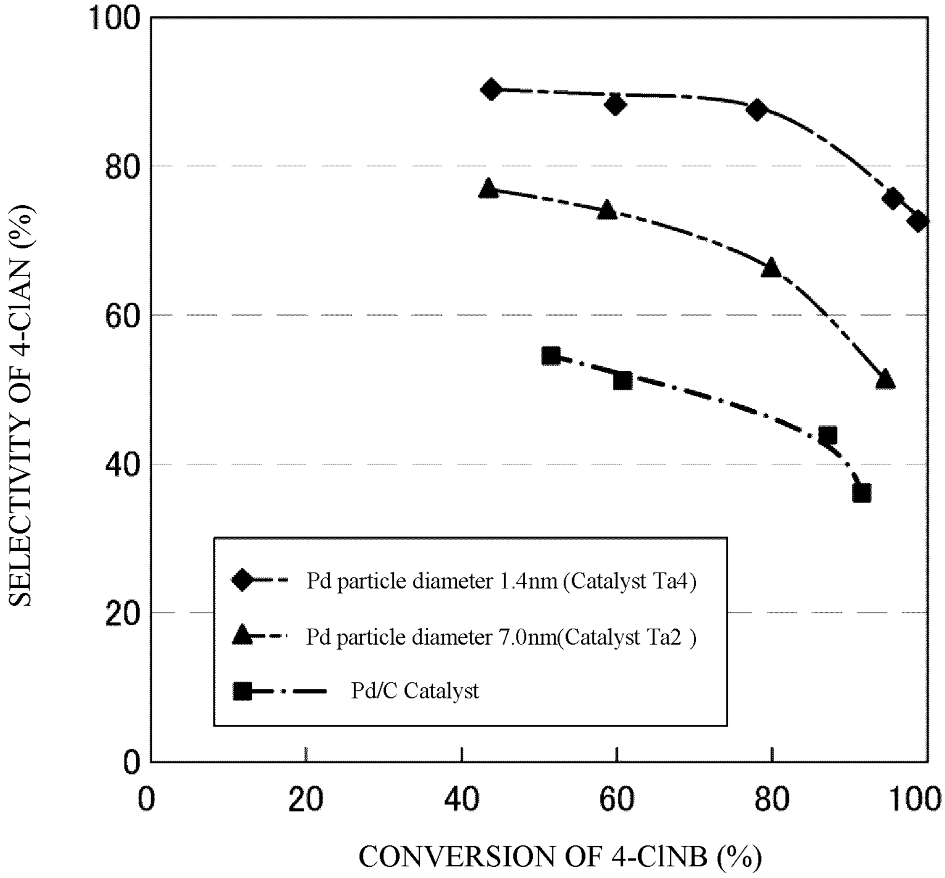
SELECTIVITY OF 4-ClAN (%)
CONVERSION OF 4-ClNB (%)
Pd particle diameter 1.4nm (Catalyst Ta4)
Pd particle diameter 7.0nm(Catalyst Ta2 )
Pd/C Catalyst
0
20
40
60
80
100

Fig.9B

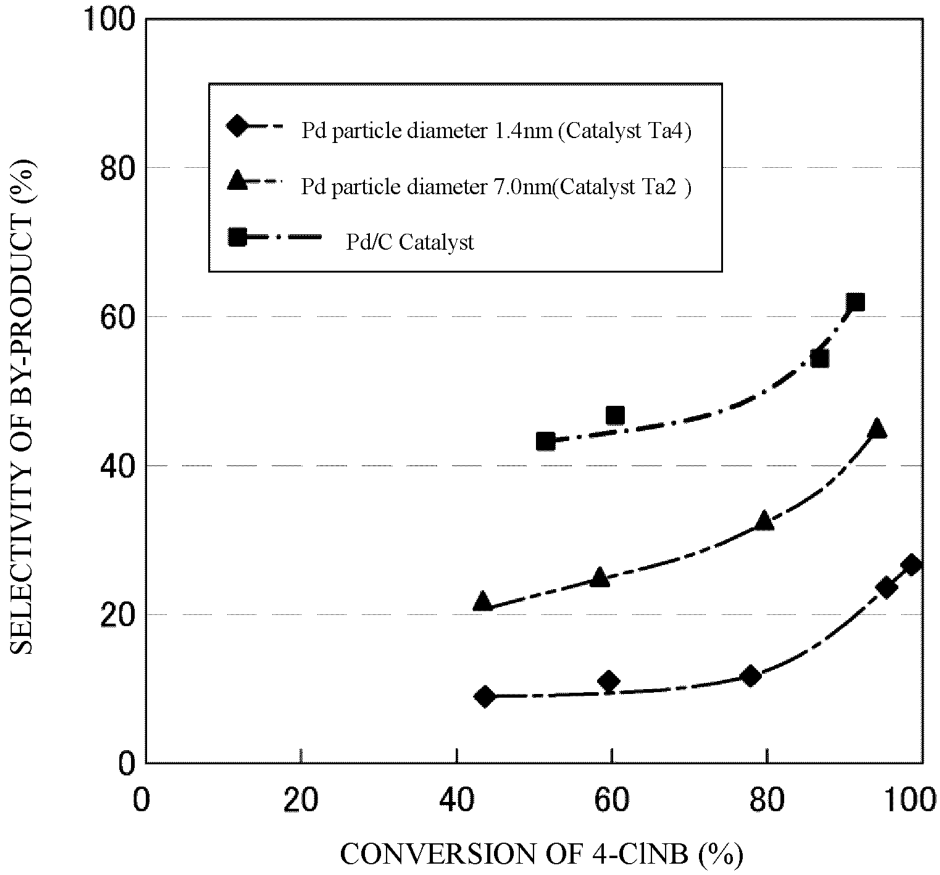
SELECTIVITY OF BY-PRODUCT (%)
CONVERSION OF 4-ClNB (%)
Pd particle diameter 1.4nm (Catalyst Ta4)
Pd particle diameter 7.0nm(Catalyst Ta2 )
Pd/C Catalyst
0
20
40
60
80
100

Fig.10A

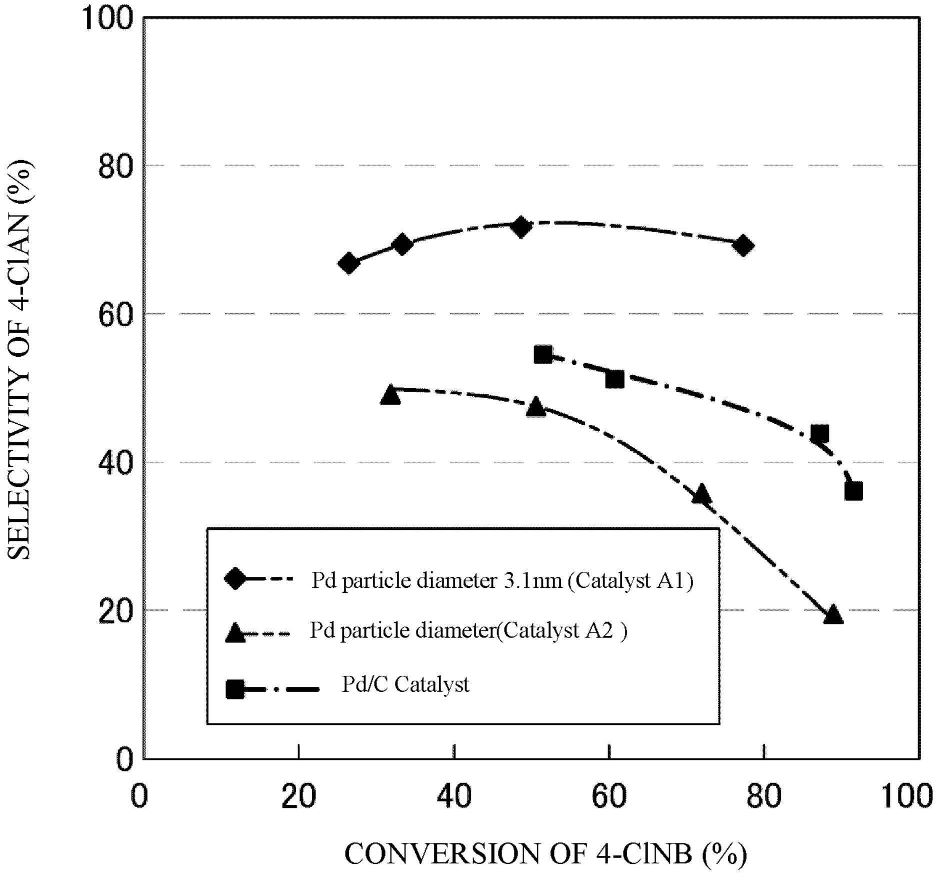


Fig.10B

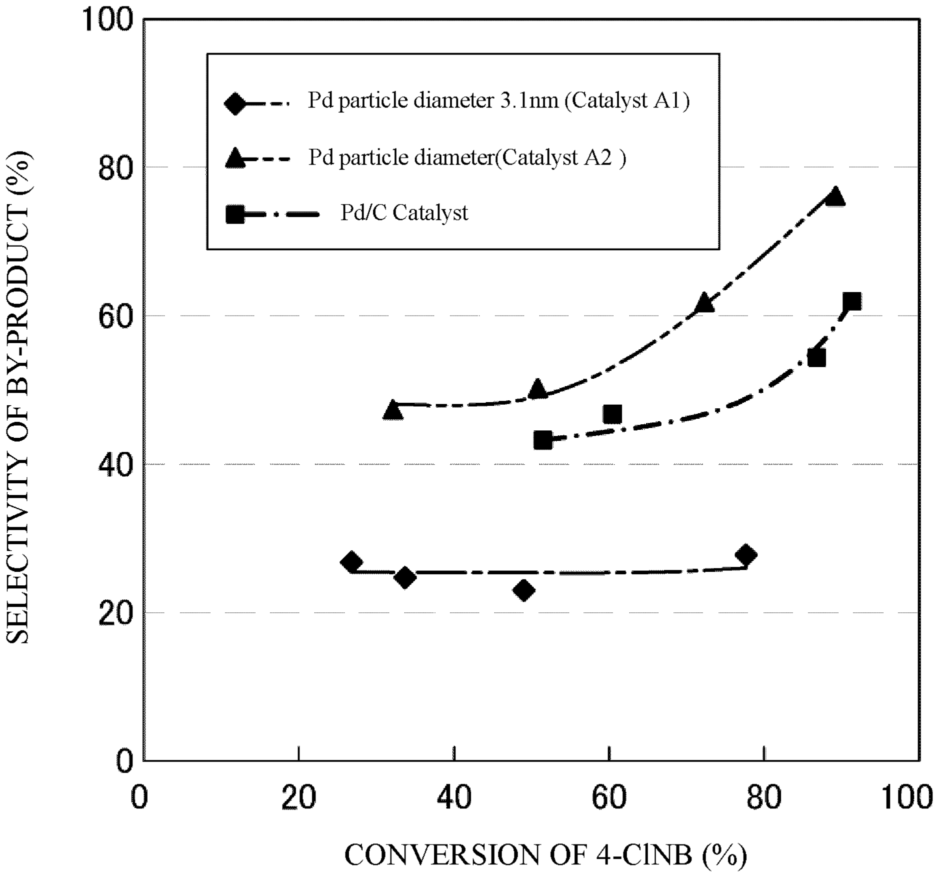

Fig.11A

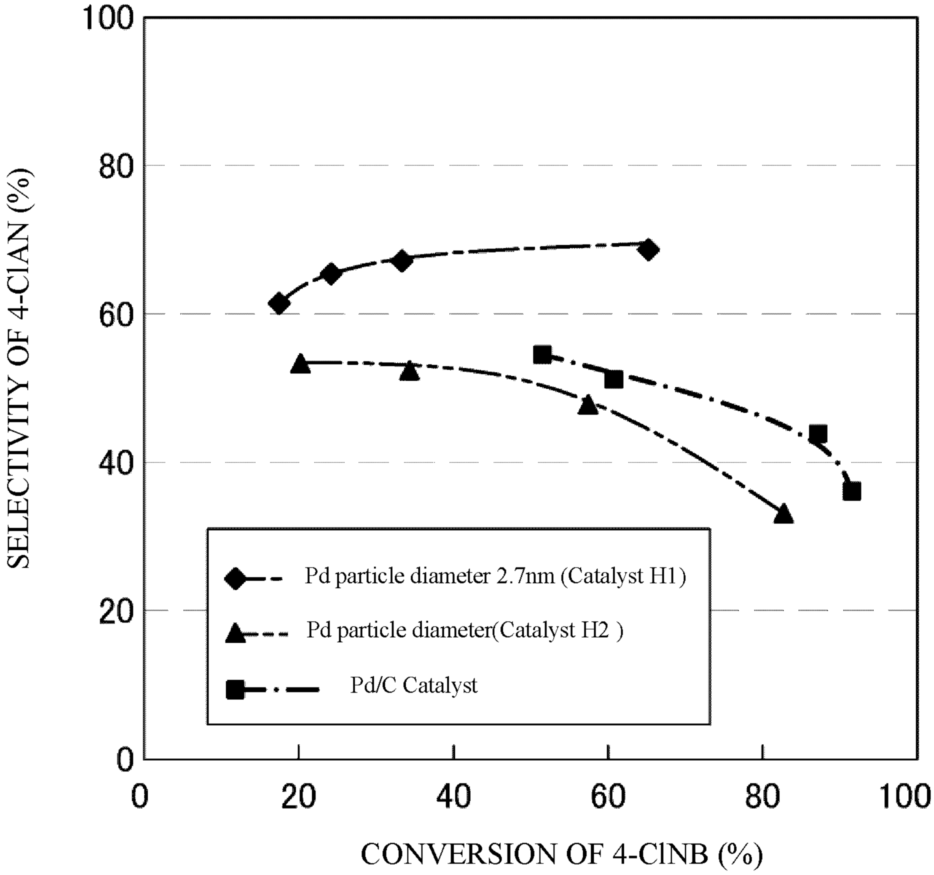


Fig.11B

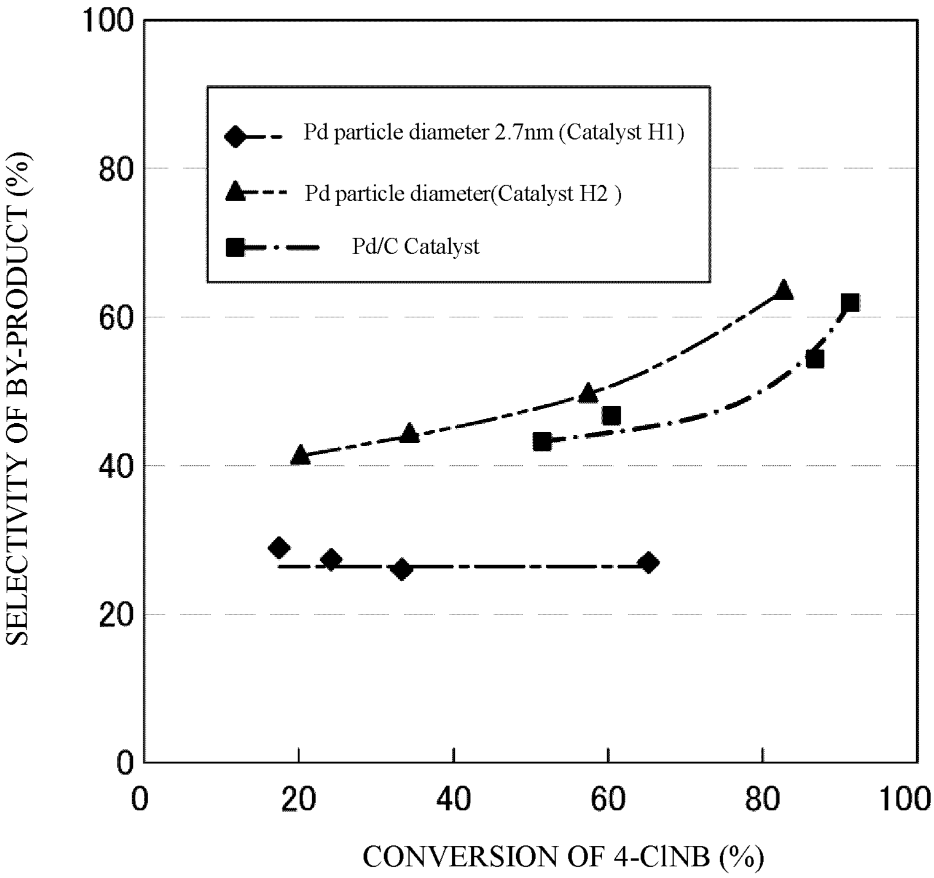

Fig.12A

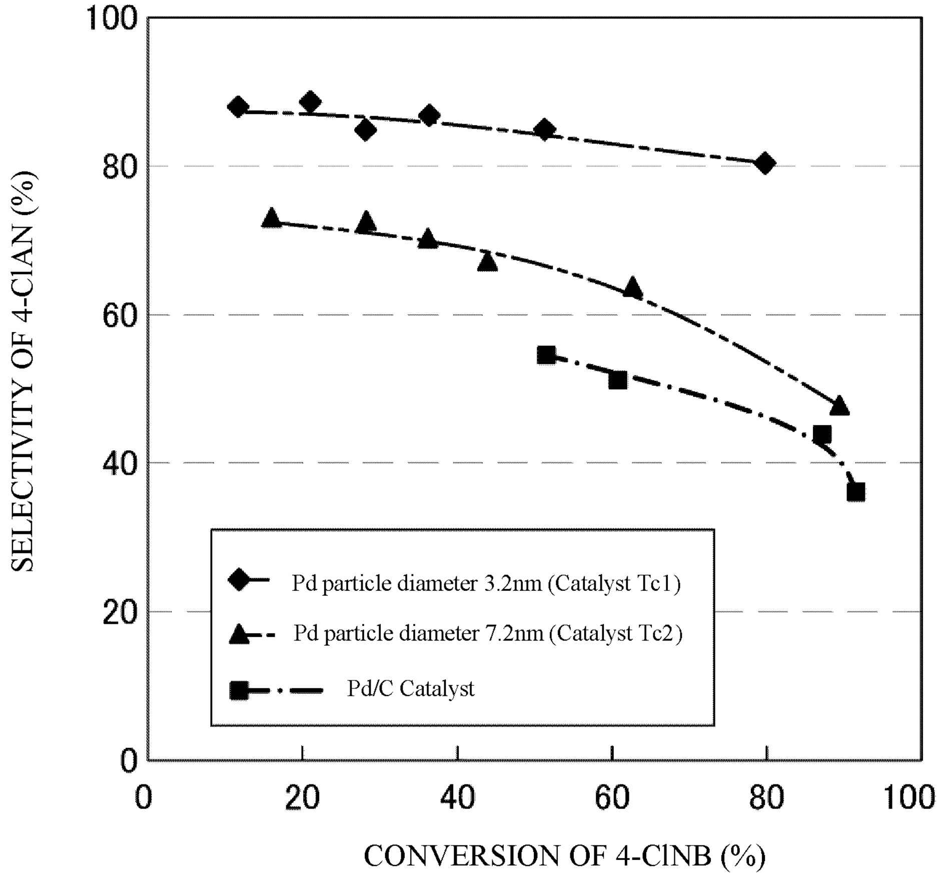


Fig.12B

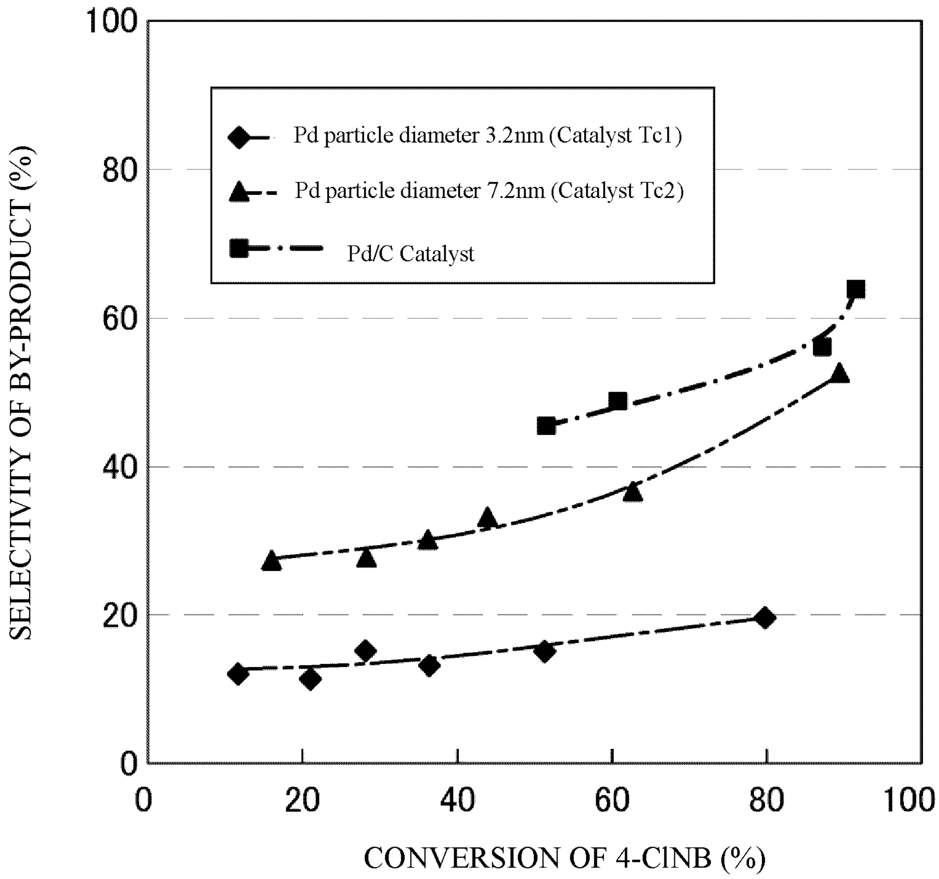

Fig.13A

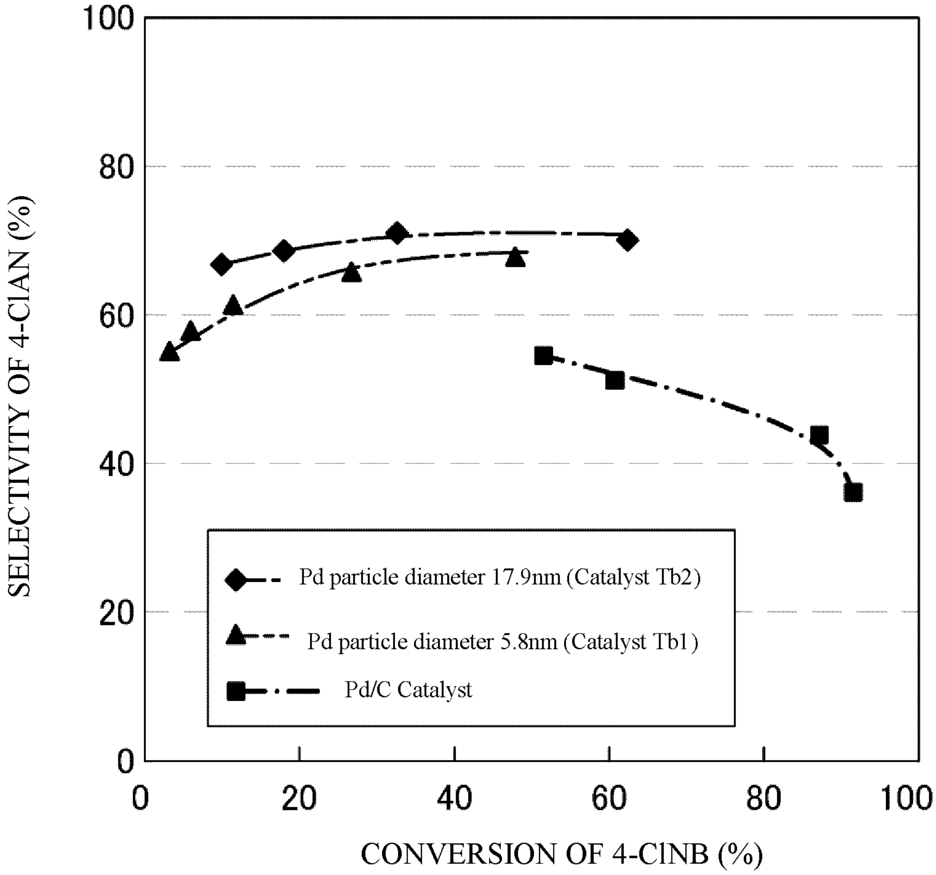


Fig.13B

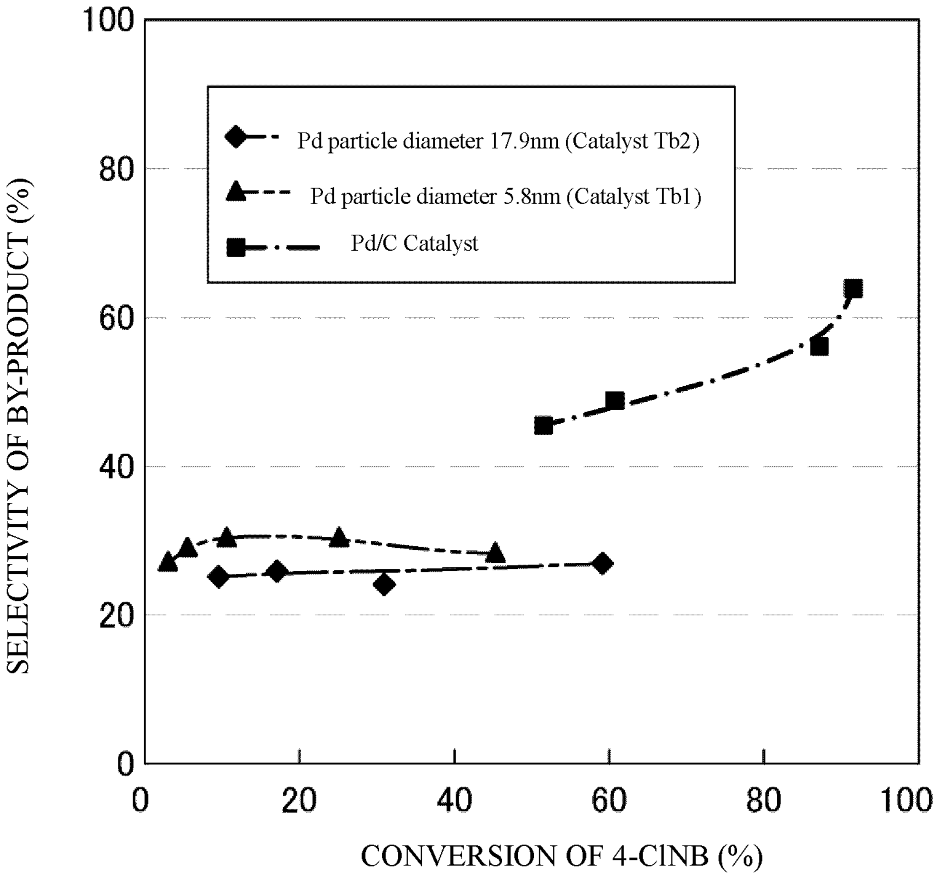

Fig.14A

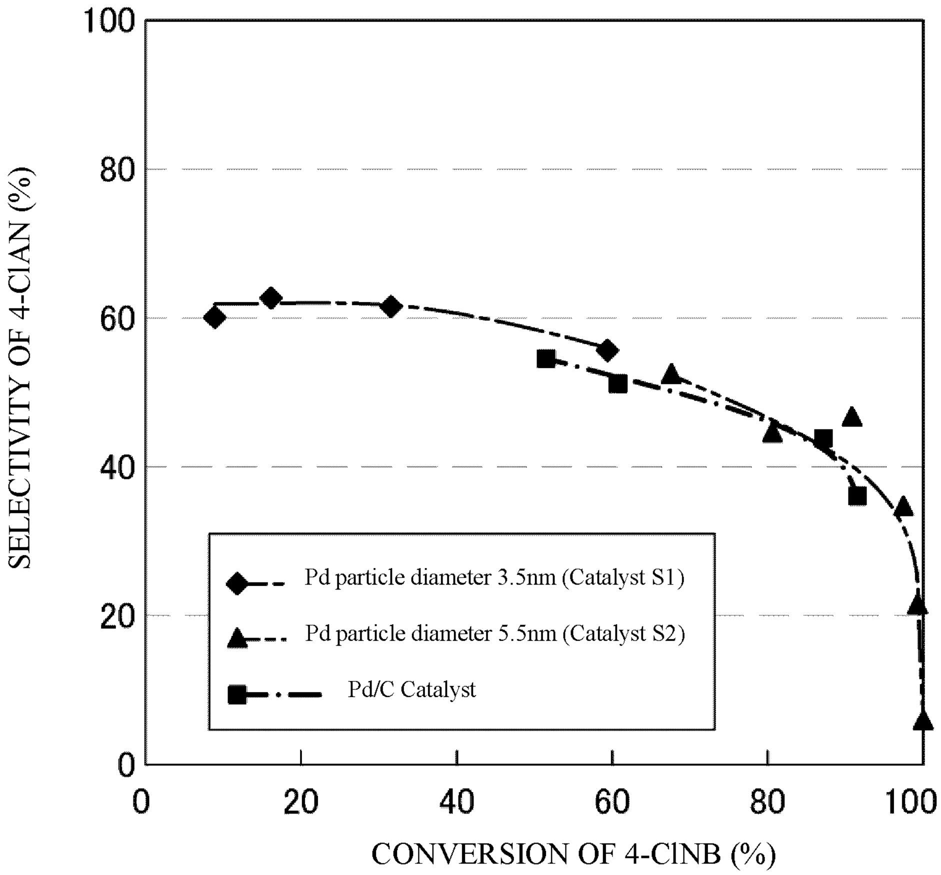


Fig.14B

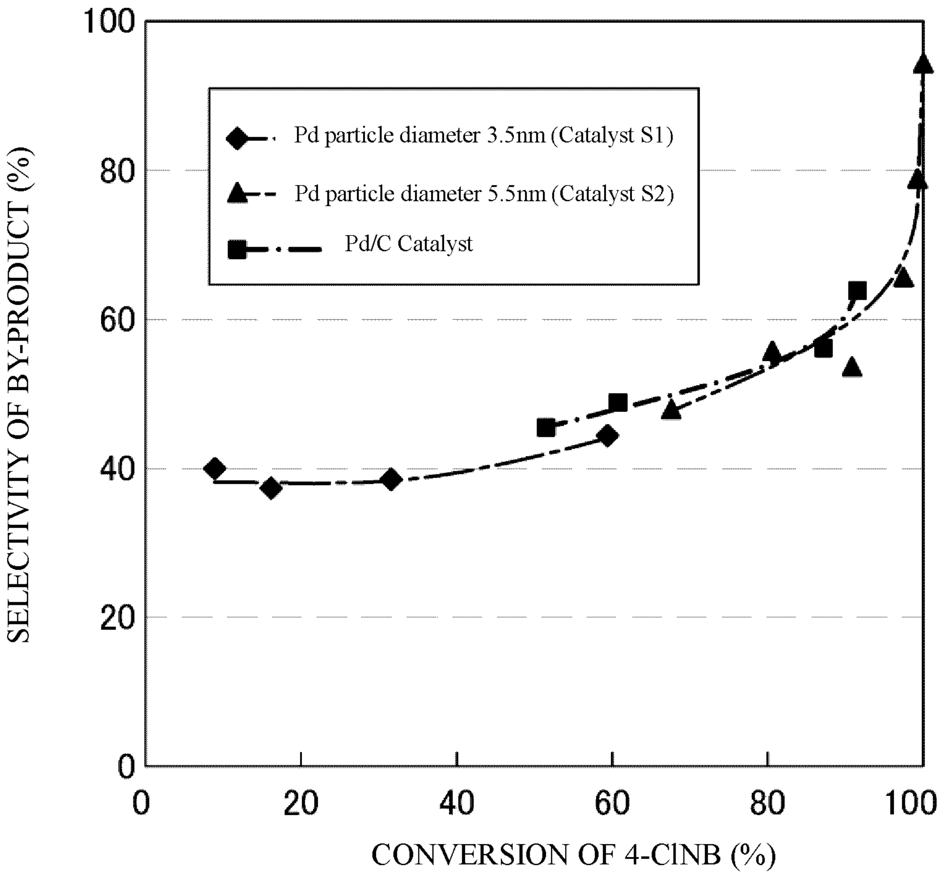

Fig.15

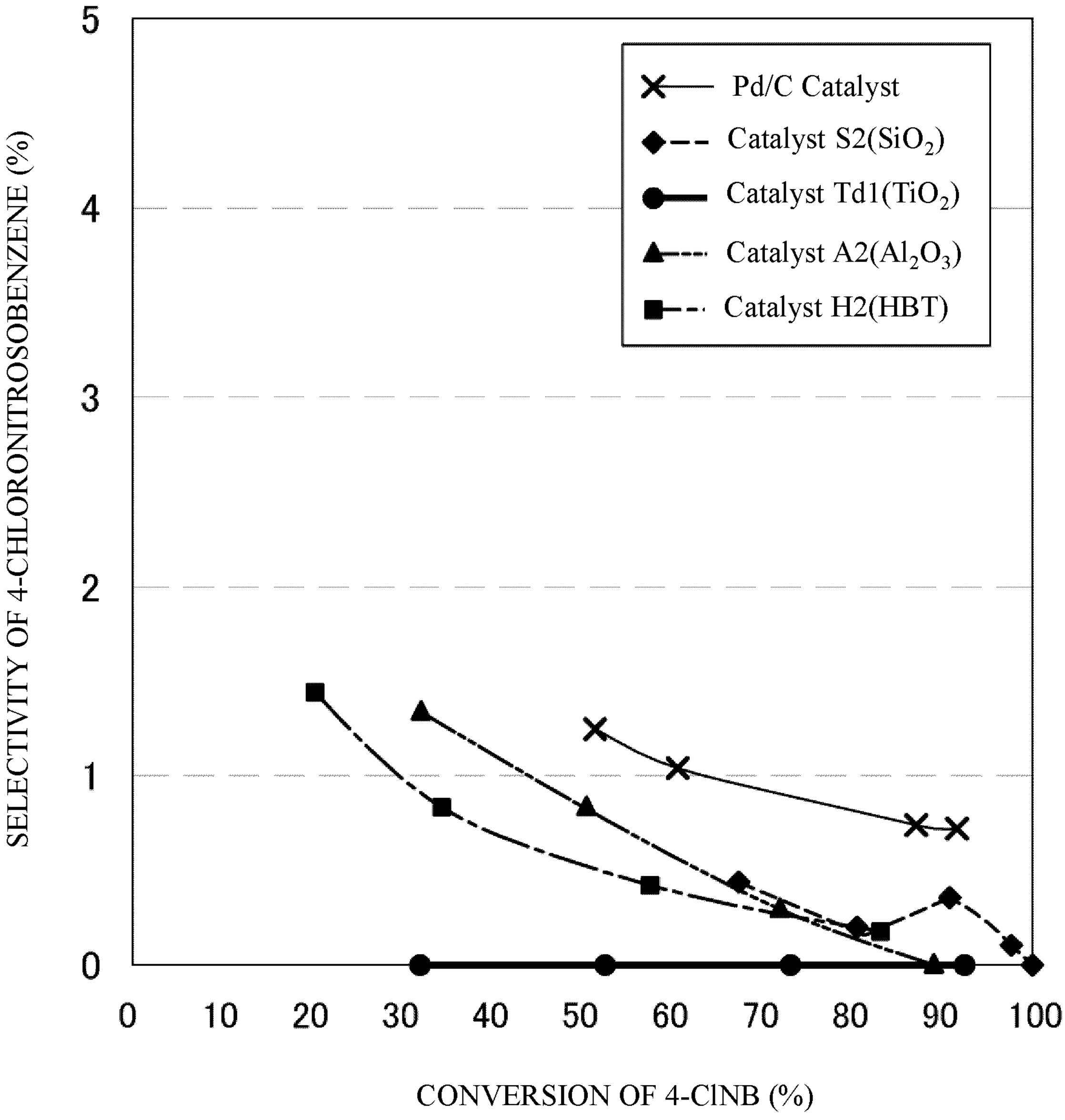
5
4
3
2
1
0
0
10
20
30
40
50
60
70
80
90
100
SELECTIVITY OF 4-CHLORONITROSOBENZENE (%)
CONVERSION OF 4-ClNB (%)
Pd/C Catalyst
Catalyst S2(SiO2)
Catalyst Td1(TiO2)
Catalyst A2(Al2O3)
Catalyst H2(HBT)

Fig.16

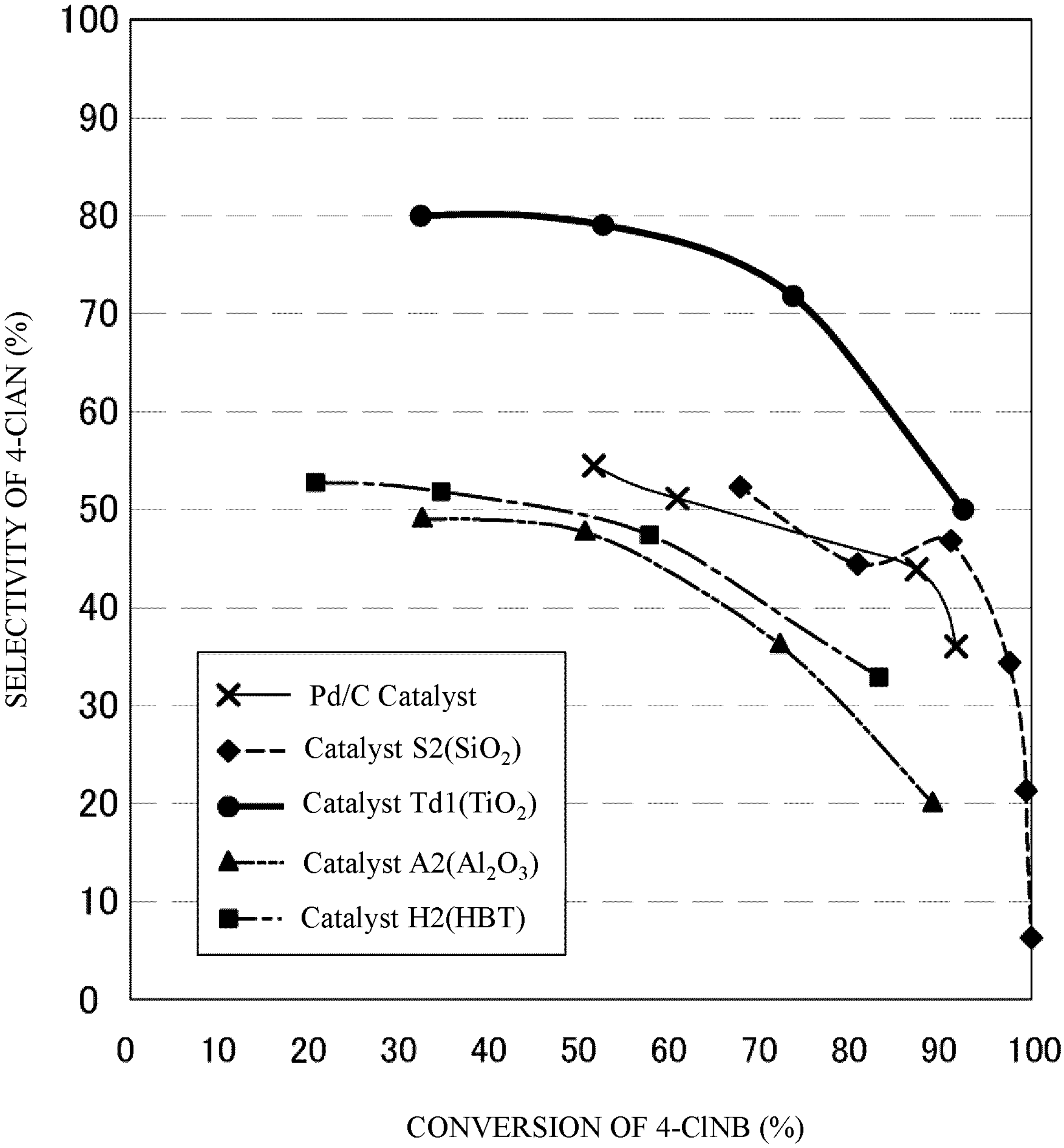
100
90
80
70
60
50
40
30
20
10
0
SELECTIVITY OF 4-ClAN (%)
0
10
20
30
40
50
60
70
80
90
100
CONVERSION OF 4-ClNB (%)
Pd/C Catalyst
Catalyst S2(SiO2)
Catalyst Td1(TiO2)
Catalyst A2(Al2O3)
Catalyst H2(HBT)

Fig.17

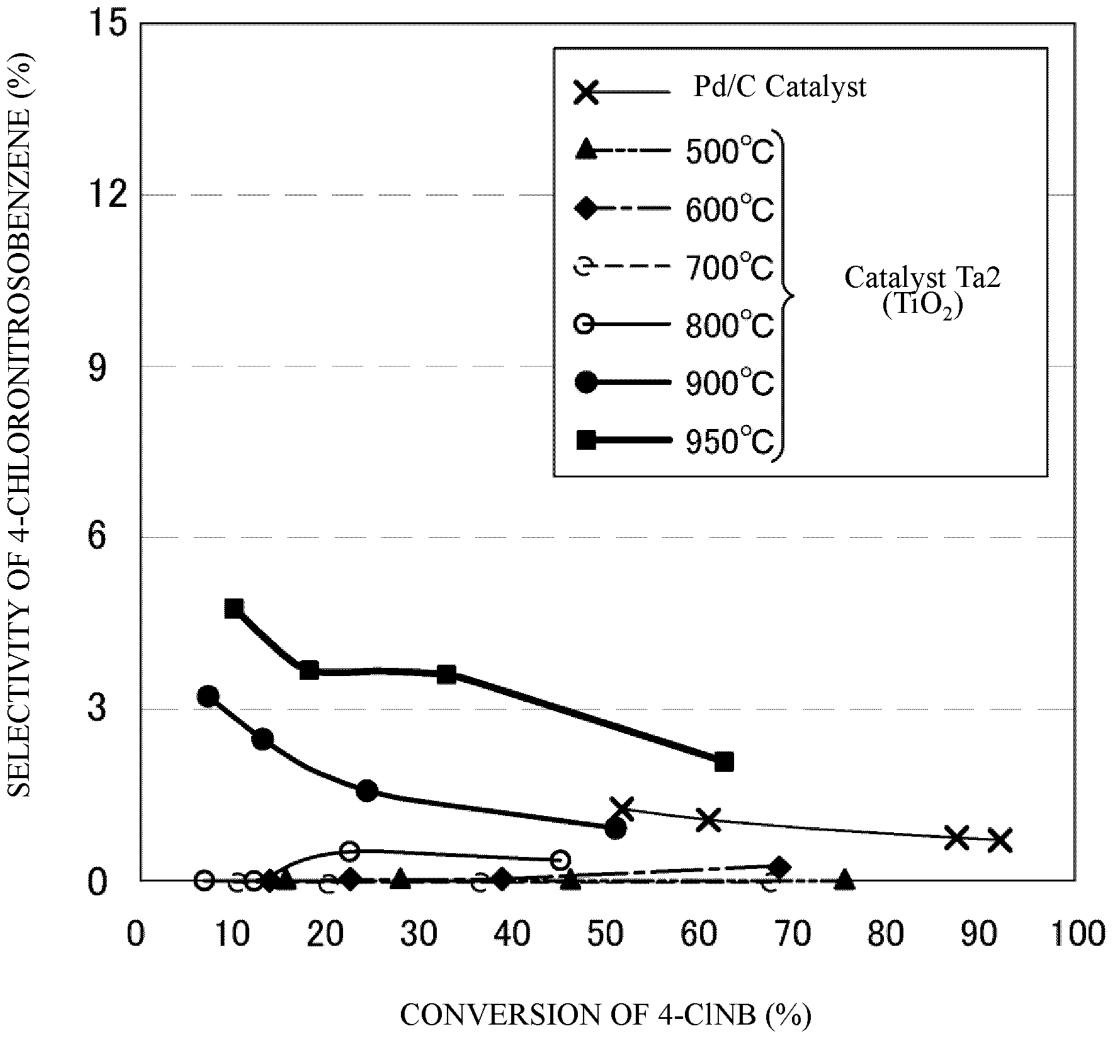
SELECTIVITY OF 4-CHLORONITROSOBENZENE (%)
15
12
9
6
3
0
0
10
20
30
40
50
60
70
80
90
100
CONVERSION OF 4-ClNB (%)
Pd/C Catalyst
500°C
600°C
700°C
800°C
900°C
950°C
Catalyst Ta2
(TiO2)

Fig.18

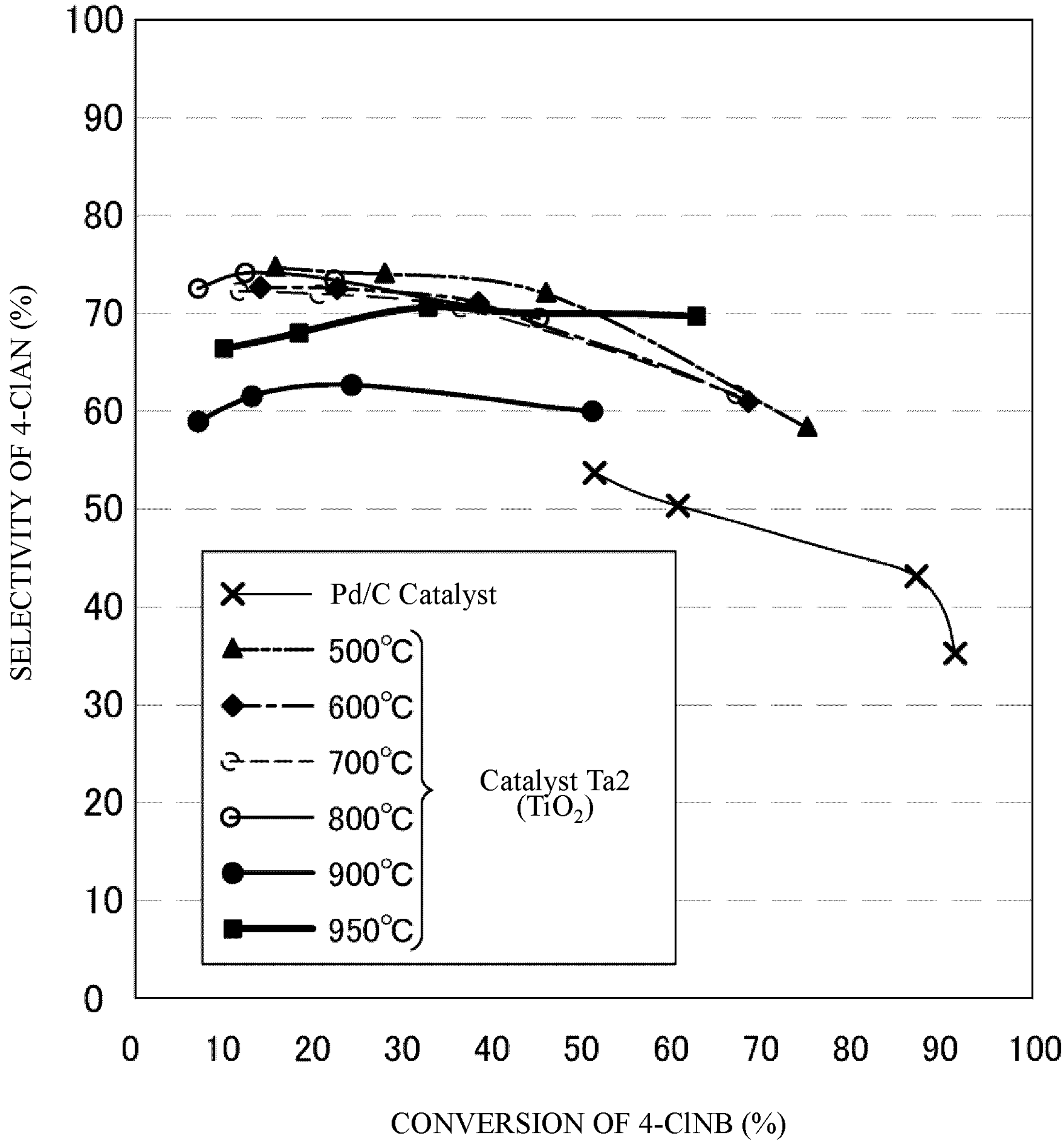
100
90
80
70
60
50
40
30
20
10
0
SELECTIVITY OF 4-ClAN (%)
0
10
20
30
40
50
60
70
80
90
100
CONVERSION OF 4-ClNB (%)
Pd/C Catalyst
500°C
600°C
700°C
800°C
900°C
950°C
Catalyst Ta2
(TiO2)

Fig.19

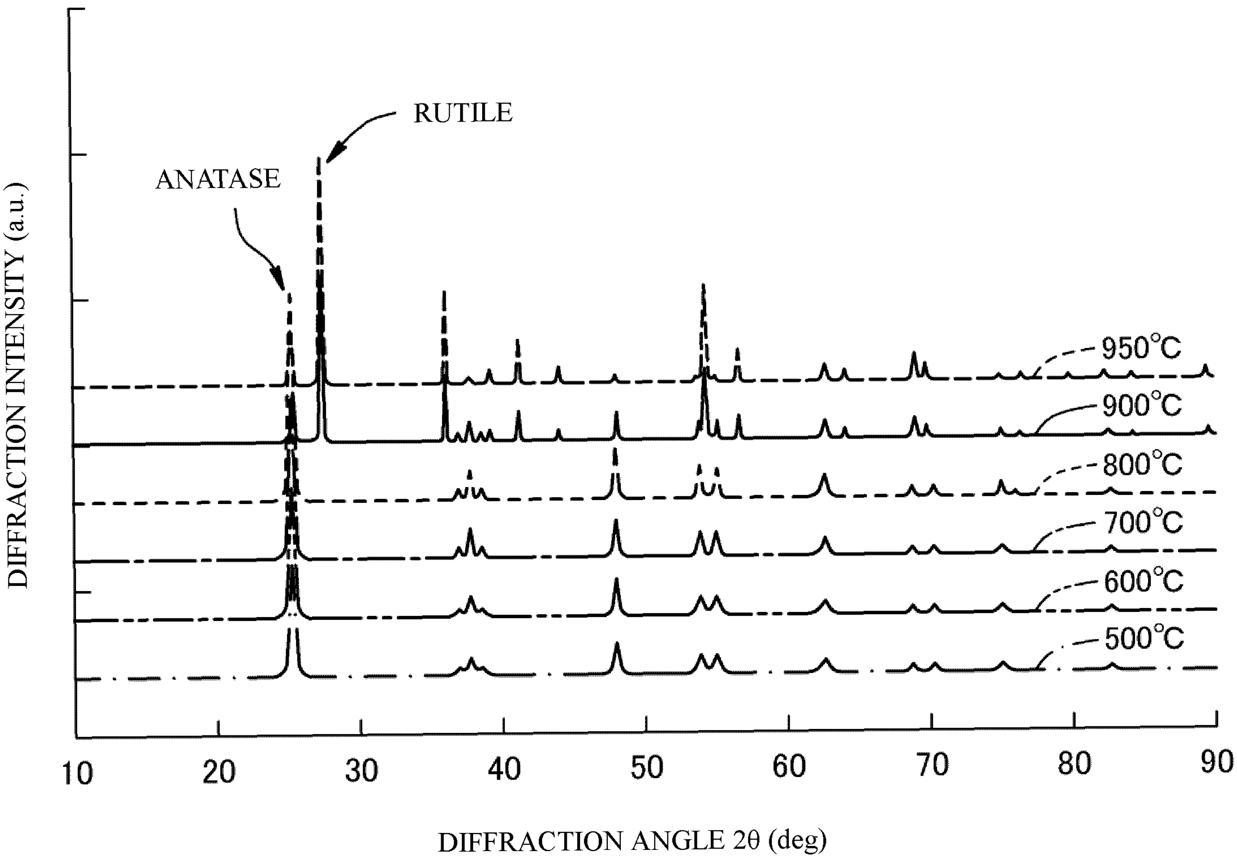
RUTILE
ANATASE
DIFFRACTION INTENSITY (a.u.)
950°C
900°C
800°C
700°C
600°C
500°C
10
20
30
40
50
60
70
80
90
DIFFRACTION ANGLE 2θ (deg)

Fig.20

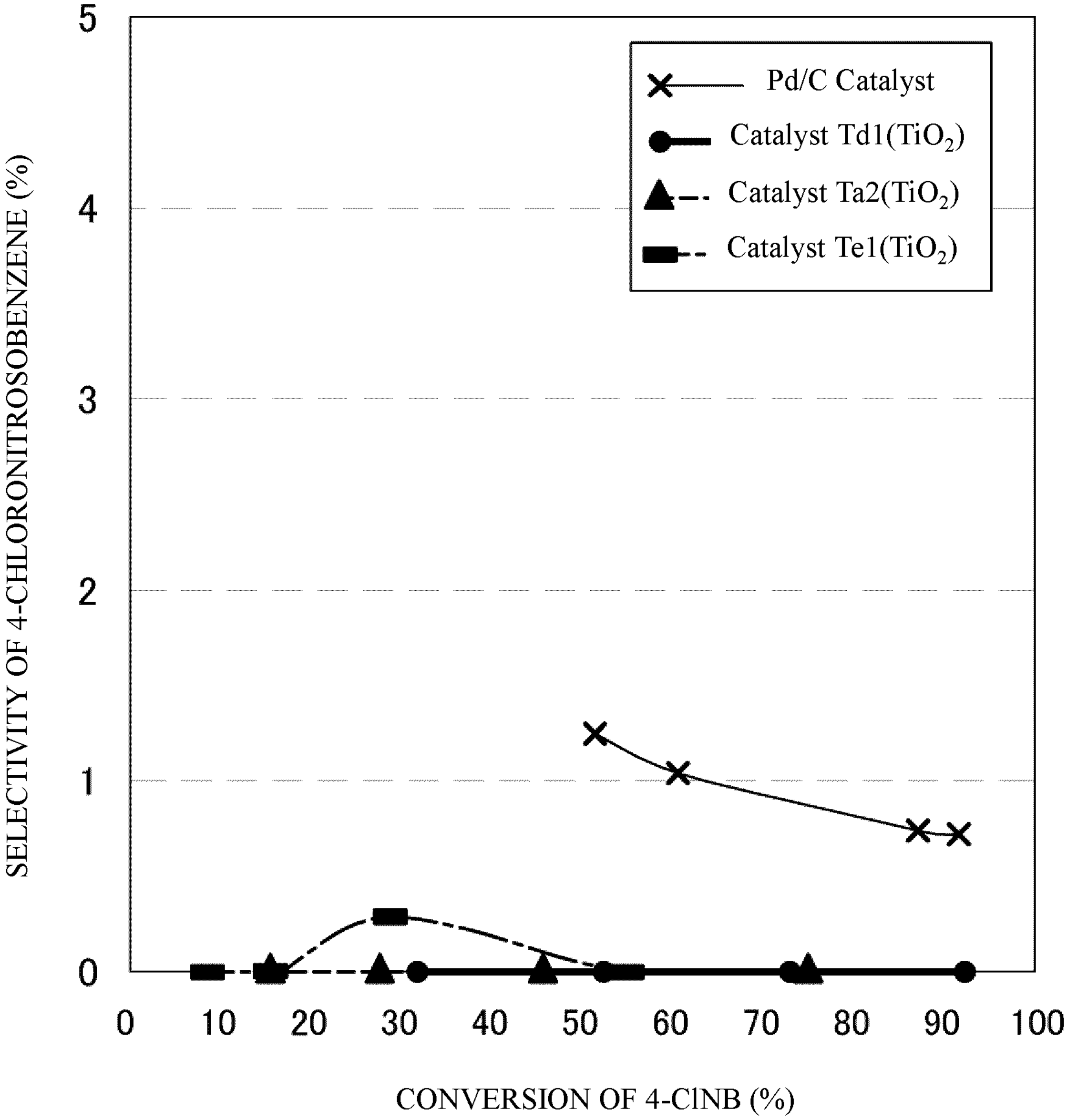
5
4
3
2
1
0
0
10
20
30
40
50
60
70
80
90
100
SELECTIVITY OF 4-CHLORONITROSOBENZENE (%)
CONVERSION OF 4-ClNB (%)
Pd/C Catalyst
Catalyst Td1(TiO2)
Catalyst Ta2(TiO2)
Catalyst Te1(TiO2)

Fig.21

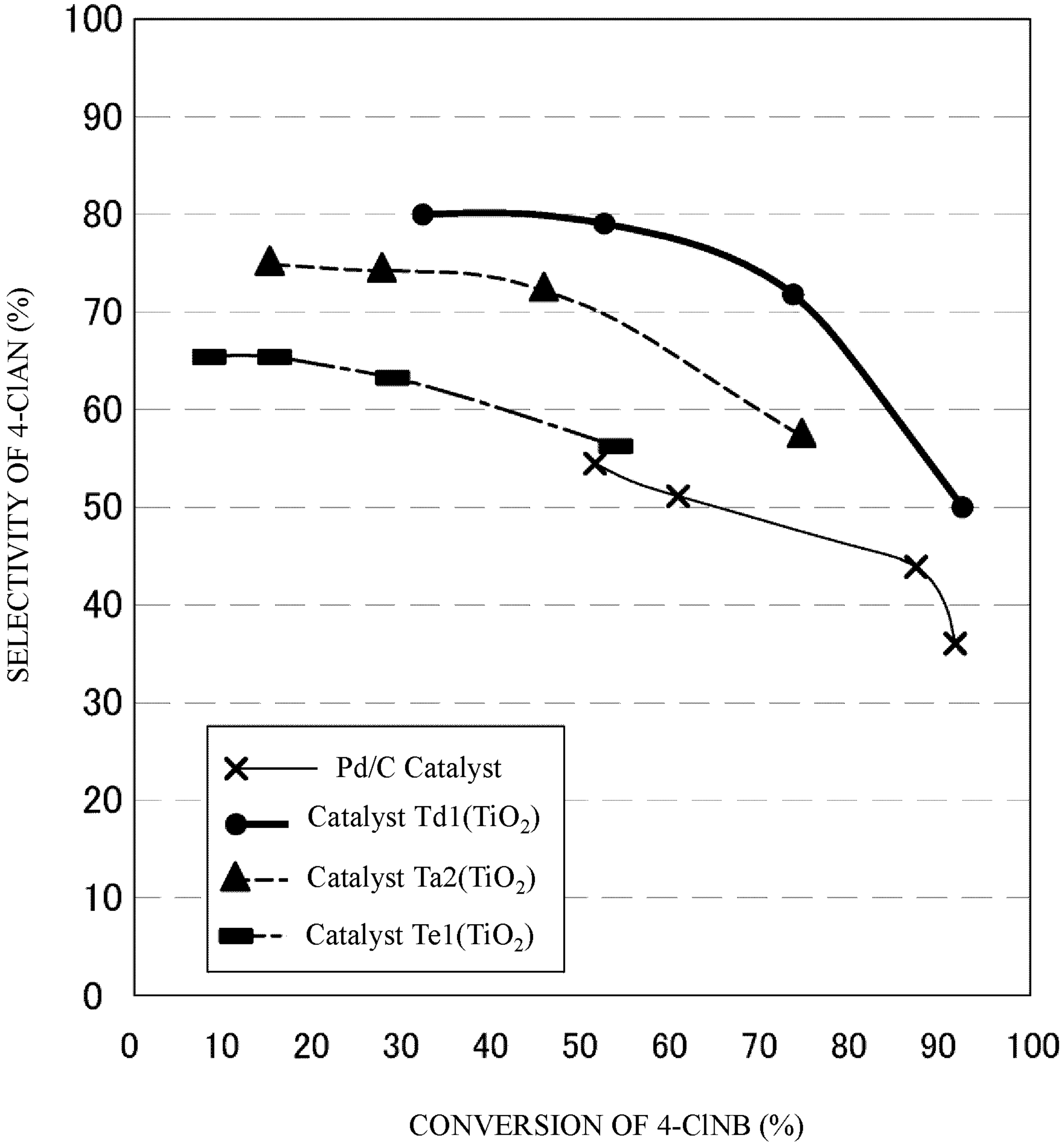
SELECTIVITY OF 4-ClAN (%)
CONVERSION OF 4-ClNB (%)
100
90
80
70
60
50
40
30
20
10
0
0
10
20
30
40
50
60
70
80
90
100
Pd/C Catalyst
Catalyst Td1(TiO2)
Catalyst Ta2(TiO2)
Catalyst Te1(TiO2)

**INTERNATIONAL SEARCH REPORT**

International application No. **PCT/JP2023/025732**

**A. CLASSIFICATION OF SUBJECT MATTER**

***B01J 23/44***(2006.01)i; ***B01J 35/10***(2006.01)i; ***C07C 209/36***(2006.01)i; ***C07C 211/52***(2006.01)i; *C07B 61/00*(2006.01)n
FI: B01J23/44 Z; B01J35/10 301G; C07C209/36; C07C211/52; C07B61/00 300

According to International Patent Classification (IPC) or to both national classification and IPC

**B. FIELDS SEARCHED**

Minimum documentation searched (classification system followed by classification symbols)

B01J21/00-38/74; C07C209/36; C07C211/52; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | SIKHWIVHILU, Lucky M. et al. Selective hydrogenation of o-chloronitrobenzene over palladium supported nanotubular titanium dioxide derived catalysts. Catalysis Communications. December 2007, vol. 8, no. 12, pp. 1999-2006, DOI: 10.1016/j.catcom.2007.03.023<br>abstract, p. 1999, left column, 1st paragraph to p. 2000, right column, 1st paragraph, p. 2000, right column, 8th paragraph to p. 2001, left column, 1st paragraph, p. 2002, right column, 2nd paragraph, p. 2006, left column, 2nd paragraph, scheme 1, fig. 9, table 1 | 1-2, 4, 6-10 |
| X | SUPRIYA, P. et al. Biomimetic synthesis of gum acacia mediated Pd-ZnO and Pd-TiO2— Promising nanocatalysts for selective hydrogenation of nitroarenes. Materials Chemistry and Physics. 09 October 2017, vol. 204, pp. 27-36, DOI: 10.1016/j.matchemphys.2017.10.026<br>abstract, p. 27, right column, 1st paragraph to p. 28, right column, 1st paragraph, p. 29, left column, 1st paragraph to p. 30, right column, 2nd paragraph, p. 32, left column, 2nd paragraph to right column, 1st paragraph, p. 33, left column, 3rd paragraph to p. 34, left column, 1st paragraph, p. 35, left column, 4th paragraph to right column, 1st paragraph, fig. 2, 4, 8, tables 1-3 | 1-2, 4-10 |
| X | JP 03-184944 A (RHONE POULENC CHIMIE) 12 August 1991 (1991-08-12)<br>claims, p. 1, lower right column, line 14 to p. 3, lower left column, line 20, example 14 | 1-2, 4-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2023** | **26 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/025732**

**C. DOCUMENTS CONSIDERED TO BE RELEVANT**

| Category* | Citation of document, with indication, where appropriate, of the relevant passages | Relevant to claim No. |
|---|---|---|
| X | CARDENAS-LIZANA, Fernando et al. Selective Gas Phase Hydrogenation of p-Chloronitrobenzene over Pd Catalysts: Role of the Support. ACS Catalysis. 02 May 2013, vol. 3, no. 6, pp. 1386-1396, DOI: 10.1021/cs4001943<br>abstract, p. 1386, left column, 1st paragraph to p. 1387, right column, 1st paragraph, p. 1388, left column, 2nd paragraph to p. 1391, left column, 1st paragraph, p. 1392, right column, 1st paragraph to p. 1394, right column, 1st paragraph, tables 1-2, fig. 2, 6, scheme 1 | 1-2, 4-10 |
| A | JP 2020-124663 A (CHIYODA CORP) 20 August 2020 (2020-08-20)<br>entire text, all drawings | 1-10 |
| A | JP 50-031134 B1 (MITSUI TOATSU CHEMICALS, INC.) 07 October 1975 (1975-10-07)<br>entire text | 1-10 |
| P, X | JP 2022-166739 A (CHIYODA CORP) 02 November 2022 (2022-11-02)<br>examples | 1-10 |

Form PCT/ISA/210 (second sheet) (January 2015)

**INTERNATIONAL SEARCH REPORT**
**Information on patent family members**

International application No.
**PCT/JP2023/025732**

| Patent document cited in search report | Publication date (day/month/year) | Patent family member(s) | Publication date (day/month/year) |
|---|---|---|---|
| JP 03-184944 A | 12 August 1991 | EP 421878 A1<br>FR 2652576 A1<br>PT 95498 A<br>DD 299175 A<br>CA 2026830 A<br>IE 903547 A | |
| JP 2020-124663 A | 20 August 2020 | (Family: none) | |
| JP 50-031134 B1 | 07 October 1975 | (Family: none) | |
| JP 2022-166739 A | 02 November 2022 | WO 2022/224741 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S51122029 A
- JP S5235652 B
- JP H7133255 A
- JP 2004277409 A
- JP S63033902 B
- JP 2021072144 A
- JP 4119144 B
- JP 6456204 B
- JP 5599212 B


### Non-patent literature cited in the description


- Strong Interactions between Pt Single Atoms and TiO. *Angew. Chem. Int. Ed.*, 2020, vol. 59, 11824-11829